(19) Europäisches Patentamt / European Patent Office / Office européen des brevets

(11) **EP 3 198 011 B1**

(12) **EUROPÄISCHE PATENTSCHRIFT**

(45) Veröffentlichungstag und Bekanntmachung des Hinweises auf die Patenterteilung:
**03.04.2019 Patentblatt 2019/14**

(21) Anmeldenummer: **15777887.9**

(22) Anmeldetag: **22.09.2015**

(51) Int Cl.:
**C12N 15/10** (2006.01)  **C12Q 1/68** (2018.01)

(86) Internationale Anmeldenummer:
**PCT/EP2015/071662**

(87) Internationale Veröffentlichungsnummer:
**WO 2016/046168 (31.03.2016 Gazette 2016/13)**

(54) **VERFAHREN ZUM NACHWEIS VON NUKLEINSÄUREN IN PROBEN MIT BIOLOGISCHEM MATERIAL**

METHOD FOR DETECTING NUCLEIC ACIDS IN SAMPLES WITH BOLOGICAL MATERIAL

PROCÉDÉ PERMETTANT DE DÉCELER DES ACIDES NUCLÉIQUES DANS DES ÉCHANTILLONS CONTENANT DU MATÉRIAU BIOLOGIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.09.2014 LU 92552**

(43) Veröffentlichungstag der Anmeldung:
**02.08.2017 Patentblatt 2017/31**

(73) Patentinhaber: **Glycotope GmbH**
**13125 Berlin (DE)**

(72) Erfinder:
• **GOLETZ, Steffen**
  **16548 Glienicke-Nordbahn (DE)**
• **WEIGELT, Doreen**
  **13125 Berlin (DE)**
• **TROESTER, Helmut**
  **68165 Mannheim (DE)**
• **VOGT, Jana**
  **69115 Dossenheim (DE)**

(74) Vertreter: **Schiweck Weinzierl Koch**
**Patentanwälte Partnerschaft mbB**
**Ganghoferstraße 68 B**
**80339 München (DE)**

(56) Entgegenhaltungen:
**US-A1- 2005 069 902**

• **XIN YU ET AL: "Development of a direct DNA extraction protocol for real-time PCR detection of Giardia lamblia from surface water", ECOTOXICOLOGY, KLUWER ACADEMIC PUBLISHERS, BO, Bd. 18, Nr. 6, 5. Juni 2009 (2009-06-05), Seiten 661-668, XP019674118, ISSN: 1573-3017**
• **CARROLL M L ET AL: "Large-scale analysis of the Alu Ya5 and Yb8 subfamilies and their contribution to human genomic diversity", JOURNAL OF MOLECULAR BIOLOGY, ACADEMIC PRESS, UNITED KINGDOM, Bd. 311, Nr. 1, 3. August 2001 (2001-08-03), Seiten 17-40, XP004469265, ISSN: 0022-2836, DOI: 10.1006/JMBI.2001.4847**
• **HU B ET AL: "Optimization and validation of DNA extraction and real-time PCR assay for the quantitative measurement of residual host cell DNA in biopharmaceutical products", JOURNAL OF PHARMACEUTICAL AND BIOMEDICAL ANALYSIS, Bd. 88, 30. August 2013 (2013-08-30), Seiten 92-95, XP028783014, ISSN: 0731-7085, DOI: 10.1016/J.JPBA.2013.08.027**
• **PEPER GRIT ET AL: "Direct real-time quantitative PCR for measurement of host-cell residual DNA in therapeutic proteins", JOURNAL OF PHARMACEUTICAL AND BIOMEDICAL ANALYSIS, Bd. 100, 12. August 2014 (2014-08-12), Seiten 123-130, XP029067075, ISSN: 0731-7085, DOI: 10.1016/J.JPBA.2014.07.032**

**(Forts. nächste Seite)**

• YANG D Y ET AL: "TECHNICAL NOTE: IMPROVED DNA EXTRACTION FROM ANCIENT BONES USING SILICA-BASED SPIN COLUMNS", AMERICAN JOURNAL OF PHYSICAL ANTHROPO, JOHN WILEY & SONS, INC, US, vol. 105, no. 4, 1 April 1998 (1998-04-01) , pages 539-543, XP008000703, ISSN: 0002-9483, DOI: 10.1002/(SICI)1096-8644(199804)105:4<539:: AID-AJPA10>3.0.CO;2-1

## Beschreibung

### Hintergrund

Biopharmazeutische Produkte werden durch Fermentation entweder mittels mikrobieller oder eukaryotischer Wirtszellen in teilweise komplexen Medien hergestellt. Zubereitungen von Wirkstoffen aus solchen Fermentationen enthalten regelmäßig bei der Ernte, neben dem gewünschten Produkt, eine Reihe von biologischen Molekülen aus den Wirtszellen, die als Verunreinigungen vorhanden sind. Zu diesen Verunreinigungen gehören neben Wirtszell-DNA auch Fette, Kohlenhydrate oder Bestandteile der bakteriellen oder pilzlichen Zellwand oder der eukaryotischen Zellmembran.

Diese Verunreinigungen werden oftmals bei der Isolation und/oder der Reinigung des gewünschten Produkts, sei es aus Wirtszellen oder der Fermentationsbrühe, z.B. dem Überstand, mitisoliert und/oder mitgereinigt. Es ist zudem bekannt, dass aus manchen Wirtszellen stammende biologische Moleküle toxische Wirkung aufweisen können. Daher ist die Entfernung der Wirtszelle und aller sonstigen verunreinigenden Materialien wünschenswert, um mögliche nachteilige Auswirkungen zu verhindern. Die vollständige Entfernung aller von Wirtszellen stammenden Verunreinigungen ist technisch anspruchsvoll und teilweise schwierig, aber aus regulatorischen Gründen vorgeschrieben.

Regulierungsbehörden für die Zulassung von Lebensmitteln, Diagnostika oder Arzneimitteln definieren daher noch zulässige Werte von Nebenprodukten bei biotechnologisch hergestellten Produkten, darunter Grenzwerte für Wirtszell-DNA in solchen Produkten. Die behördliche Lebensmittelüberwachungs- und Arzneimittelzulassungsbehörde der Vereinigten Staaten (US Food and Drug Administration (FDA)) gibt eine obere Grenze von 100 pg pro therapeutischer Dosis an Wirtszell-DNA an (US Food and Drug Administration (1997) *Points to consider in the manufacture and testing of monoclonal antibody products for human use*), was z.B. bei der Verabreichung von therapeutischen Antikörpern in größeren Dosen relevant ist, da solche Antikörper oftmals mittel intravenöser Gabe in einem größeren Volumen verabreicht werden. Auch die Weltgesundheitsorganisation (WHO) hat zur oberen Grenze von bis zu 10 ng/Dosis Wirtszell-DNA Leitlinien veröffentlicht (World Health Organization, Technical Reports (1987), Report of a WHO Study group. *Acceptability of cell substrates for production of biologicals*.).

Hersteller biotechnologisch erzeugter Produkte, wie z.B. Arzneimittel, Diagnostika oder Lebensmittel sind verpflichtet, eine Angabe darüber zu machen, ob die im Produkt enthaltene restliche Wirtszell-DNA innerhalb der noch akzeptablen Grenzen für eine bestimmtes Produkt liegt.

Es sind verschiedene Verfahren zum Nachweis oder der Mengenbestimmung von Wirtszell-DNA verfügbar, wie z.B. die PicoGreen Analyse, Hybridisierungsanalyse, oder quantitative PCR (Mehta and Keer (2007), BioProcess International: 44-58). Obwohl die derzeit verfügbaren Verfahren zur Bestimmung von Wirtszell-DNA die gesetzlichen Anforderungen und Sicherheitsanforderungen erfüllen, gibt es einen wachsenden Bedarf in der Industrie für detaillierte Informationen über ihre Leistungsmerkmale, insbesondere ist es wünschenswert sensitivere Verfahren zur Verfügung zu haben, die in der Lage sind, auch geringste Spuren an Nukleinsäuren im Picogramm- bis Femtogrammbereich in Proben quantitativ nachweisen zu können, die biologisches Material enthalten. Solche Verfahren sind nicht nur für den Nachweis von Wirtszell-DNA in biotechnologisch erzeugten Produkten von Interesse, sondern generell für den Nachweis von Nukleinsäuren in einer Probe, die biologisches Material enthält, um z.B. in der Forensik eingesetzt zu werden.

Es ist daher die Aufgabe der vorliegenden Erfindung, ein kostengünstiges, sensitives Verfahren zum Nachweis einer Nukleinsäure in einer Probe, die biologisches Material enthält, bereitzustellen. Diese Aufgabe wird gelöst durch die in den Ansprüchen und in der folgenden Beschreibung enthaltenen Ausführungsformen und Gegenstände, die von den Beispielen und Abbildungen illustriert werden.

Hu, B. et al., J Pharm Biomed Anal.(2014), 88:92-95 und Peper, G. et al. J Pharm Biomed Anal (2014), 100:123-130 offenbaren qPCR-Nachweisverfahren für Wirtszell-DNA-Rückständen in biopharmazeutischen Produkten insbesondere therapeutischen Antikörpern bzw. in therapeutischen Proteinen.

### Detaillierte Beschreibung

Die Erfinder der vorliegenden Erfindung konnten die gestellte Aufgabe zu ihrer eigenen Überraschung so lösen, dass nunmehr ein Verfahren zum Nachweis einer Nukleinsäure in einer Probe, die biologisches Material enthält, bereitgestellt wird, das vorteilhafterweise die bisherigen unteren Grenzen durchbricht. Das erfindungsgemäße Verfahren und das Kit stoßen dabei vorzugsweise in Bereiche von lediglich 0,08 - 2,75 fg/µl Probe vor und liegt damit unterhalb der Nachweisgrenze der als sehr sensitiv bekannten quantitativen PCR (qPCR), die bei 10 fg bakterieller DNA und 5 pg Säuger DNA liegt. Dadurch ermöglicht das erfindungsgemäße Verfahren und das Kit vorteilhafterweise, dass die von Zulassungsbehörden, z.B. der FDA geforderte Obergrenze von 100 pg Wirtszell-DNA pro therapeutischer Dosis eines biotechnologisch erzeugten Produkts, insbesondere z.B. eines Antikörpers auch bei einer Verabreichung in größeren Dosierungen eingehalten werden kann. Insbesondere werden z.B. Antikörper in einem größeren Volumen von z.B. 200 ml verabreicht, so dass pro mL lediglich 0,5 pg Wirtszell-DNA enthalten sein dürfen. Herkömmliche Verfahren konnten diese untere Grenze insofern nicht unterschreiten, als deren untere Nachweisgrenze 0,5 pg Wirtszell-DNA nicht unterschreitet, so dass nicht gezeigt werden kann, dass ein biotechnologisch hergestelltes Produkt zur Verabreichung an Menschen weniger als 0,5 pg Wirtszell-DNA enthält.

Insbesondere stellten die Erfinder zu ihrer Überraschung fest, dass die Abfolge der Schritte (a) Protease-Verdau der Probe, (b) Konzentrieren der Probe, (c) Anreichern der Nukleinsäure aus der Probe und (d) Nachweisen der Nukleinsäure mittels quantitativer Polymerase Kettenreaktion (qPCR) die Nachweisgrenze kommerzieller, z.B. in der Forensik eingesetzter Kits zum Nachweis von Nukleinsäuren durchbricht, obgleich diese Kits bereits optimiert sind (siehe Beispiel 4). Des Weiteren stellten die Erfinder fest, dass die Schritte (a), (b) und (c) entscheidend sind, um eine Nukleinsäure in einer Probe in einer Menge unter 0,5 pg/ml nachweisen zu können. Weder die Abfolge der Schritte des erfindungsgemäßen Verfahrens noch die Feststellung, dass die Schritte (a), (b) und (c) entscheidend sind, um eine Nukleinsäure in einer Probe in einer Menge von unter 0,5 pg/ml verlässlich nachweisen zu können, waren im Stand der Technik bekannt oder vorgeschlagen.

Dementsprechend betrifft die vorliegende Erfindung ein Verfahren zum Nachweis einer Nukleinsäure in einer Probe, die biologisches Material enthält, umfassend

(a) Behandeln der Probe mit Protease;
(b) Konzentrieren der Probe;
(c) Anreichern der Nukleinsäure aus der Probe; und
(d) Nachweisen der Nukleinsäure mittels quantitativer PCR,

wobei das biologische Material Antikörper oder ein therapeutisches Protein enthält.

Das Verfahren ist generell ein in vitro-oder ex vivo-Verfahren.

Der Begriff "Nachweis" umfasst die Detektion sowie die Quantifizierung einer Nukleinsäure in einer Probe, die biologisches Material enthält. Das erfindungsgemäße Verfahren muss nicht zwangsweise im positiven Nachweis einer Nukleinsäure resultieren, da in einer Probe, die biologisches Material enthält, beispielsweise von Anfang an keine Nukleinsäuren enthalten sein könnte oder lediglich eine so geringe Menge an Nukleinsäuren enthalten ist, dass diese selbst mit dem erfindungsgemäßen Verfahren nicht nachweisbar ist. Der "Nachweis" mit Hilfe des erfindungsgemäßen Verfahrens umfasst daher auch das Bestimmen, ob in einer Probe, die biologisches Material enthält, eine Nukleinsäure enthalten ist oder nicht.

Dementsprechend betrifft die vorliegende Erfindung auch ein Verfahren zum Bestimmen, ob eine Probe, die biologisches Material enthält, eine Nukleinsäure enthält, umfassend

(a) Behandeln der Probe mit Protease;
(b) Konzentrieren der Probe;
(c) Anreichern der Nukleinsäure aus der Probe; und
(d) Bestimmen mittels quantitativer PCR, ob in der Probe eine Nukleinsäure enthalten ist,

wobei das biologische Material Antikörper oder ein therapeutisches Protein enthält.

Vorzugsweise wird eine Nukleinsäure mit einem erfindungsgemäßen Verfahren nicht nur qualitativ, sondern auch quantitativ wie hierin detaillierter beschrieben nachgewiesen bzw. bestimmt.

Vorzugsweise zusätzlich oder alternativ wird ein erfindungsgemäßes Verfahren eingesetzt

- zum Nachweis einer Nukleinsäure in biologischem Material (vorzugsweise einer Nukleinsäure aus einer Wirtszelle in biologischem Material), das an Menschen verabreicht wird;
- zur Bestimmung, ob biologisches Material zur Verabreichung an Menschen im Wesentlichen frei von Nukleinsäuren (insbesondere DNA), vorzugsweise frei von Wirtszell-Nukleinsäuren (insbesondere DNA ist); oder
- zum Quantifizieren von Nukleinsäuren, vorzugsweise von Wirtszell-Nukleinsäuren in einer Probe.

Bei der qPCR oder Real-Time PCR kann die PCR mitverfolgt werden. Dies erfolgt über ein Fluoreszenzsignal, welches stärker wird, je mehr PCR-Produkte (Amplikons) gebildet worden sind. Dabei ist das Fluoreszenzsignal proportional zum Gehalt des PCR-Produktes. Durch Messen des Fluoreszenzsignals, bei jedem Zyklus der PCR, kann der ansteigende Gehalt des Produktes als Kurve sichtbar gemacht werden. Durch diese Kurve können quantitative und qualitative Nukleinsäureanalysen durchgeführt werden. Eine qPCR-Amplifikationskurve kann in drei Bereiche gegliedert werden. Am Anfang übersteigt das Fluoreszenzsignal des Hintergrundes jenes der eigentlichen Amplifikation. In jedem PCR Zyklus vermehren sich die Amplikons und auch das Fluoreszenzsignal wird zunehmend stärker. Ab einem gewissen Punkt ist das Fluoreszenzsignal größer als das Hintergrundsignal. Dieser Punkt wird Crossing Point (Cp) genannt. Zu diesem Zeitpunkt beginnt auch die exponentielle Phase der qPCR Kurve. In der exponentiellen Phase befinden sich ca. 1000 amplifizierte Moleküle in einem Reaktionsgefäß. Die Quantifizierung von DNA mittels qPCR wird durch die Bestimmung des Zeitpunktes des Cp ermöglicht. Der Cp wird vorzugsweise als Zykluszahl angegeben. Zuletzt endet die Kurve in der Plateauphase. In der Plateauphase werden immer geringere Mengen an Amplikons gebildet, und schließlich stagniert die DNA-Synthese.

Die Fluoreszenzsignale der qPCR lassen sich auf verschiedene Arten erzeugen. Die am meisten genutzten Systeme sind zum einen interkalierende Fluoreszenzfarbstoffe, die an doppelsträngige DNA binden, zum anderen mit Fluoreszenz markierte Oligonukleotide, welche spezifisch an die DNA binden und erst dann eine messbare Fluoreszenz emittieren, wenn sie im Verlauf der PCR-Reaktion degradiert werden. Beispielhaft für je eine der beiden Methoden ist ein qPCR-Verfahren unter Verwendung von SYBR Green bzw. unter Verwendung von Hydrolyse Sonden.

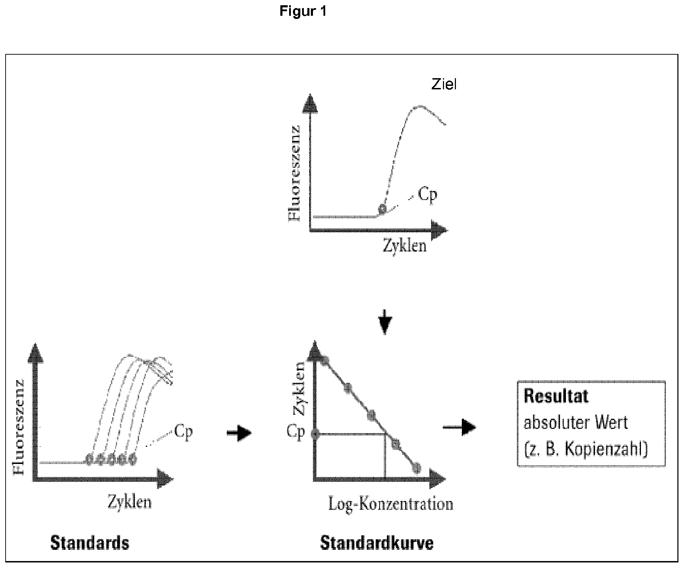

Eine Quantifizierung, die vorzugsweise im Zusammenhang mit einem erfindungsgemäßen Verfahren angewendet wird, funktioniert folgendermaßen: um die Konzentration einer Probe zu ermitteln, wird der Vergleich des Crossing Points der unbekannten Probe mit dem Cp-Wert eines zuvor definierten Standards durchgeführt. Dazu wird zunächst der Standard als Standardkurve aufgenommen. Hierzu wird eine Verdünnungsreihe des Standards angefertigt und diese per quantitativer (qPCR) vermessen. Vorzugsweise die Software ermöglicht es, die Konzentrationen des Standards anzugeben, anhand derer automatisch die Standardkurve berechnet wird. Hierbei wird der Logarithmus der Konzentrationen gegen die Cp-Werte aufgetragen. Wird nun eine unbekannte Probe gemessen, kann die Konzentration, durch Vergleich des Cp-Werts mit der Standardkurve, ermittelt werden (siehe Figur 1). Dies wird vorzugsweise automatisch durch die Software ausgeführt. Zusätzlich kann beim Messen der Probe ein Standard mitvermessen werden. Bei diesem ist die Konzentration ebenfalls bekannt, welche in das Programm eingetragen wird. Der Cp-Wert und die Konzentration des mitgemessenen Standards werden mit der Standardkurve abgeglichen und die Konzentration der unbekannten Probe anhand dieser Daten und des Cp-Wertes der Probe errechnet.

Der Standard für die Erstellung einer Standardkurve ist eine Nukleinsäure des Organismus, von dem man annimmt, das das aus dem Organismus erhaltene oder gewonnene oder von dem Organismus hergestellte biologische Material eine Nukleinsäure dieses Organismus enthält. Ein "Organismus" kann dabei ein Prokaryot, wie Bakterien oder ein Eukaryot, wie z.B. Säuger, Vogel, Fisch, Reptil, Insekt, Pilz inkl. Hefe, ein Virus oder ein Archae sein. Ein bevorzugter Organismus ist eine Wirtszelle, wie hierin definiert, die biologisches Material herstellt oder aus der biologisches Material erhalten oder gewonnen wird.

In der real-time PCR wird heute nicht mehr primär in DNA-Produktmengen oder Konzentrationen gerechnet, sondern als Maß für die Quantifizierung der Startmenge werden die sog. Ct oder Cp (= Crossing Point) Werte herangezogen. Sie entsprechen der Anzahl der PCR Zyklen, die nötig sind um ein konstant definiertes Fluoreszenzniveau zu erreichen. Am Cp befindet sich in allen Reaktionsgefäßen die gleiche Menge an neu synthetisierter DNA. Im Falle einer 100% Effizienz der PCR verdoppelt sich mit jedem Zyklus die DNA Produktmenge und analog dazu das Fluoreszenzsignal. Ein um eine Einheit geringerer Cp entspricht der doppelten Menge an eingesetzten cDNA; respektive mRNA Startmenge.

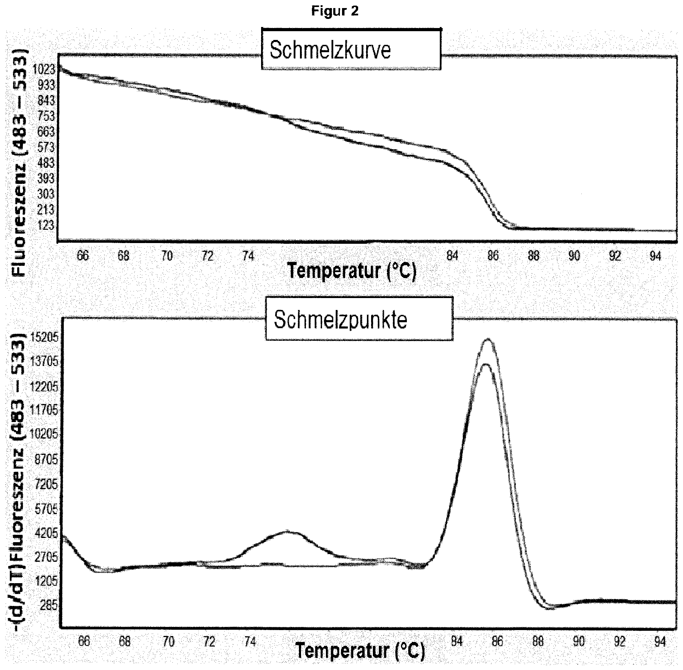

Wenn beispielsweise SYBR Green oder ein anderer, in doppelsträngige DNA interkalierender Farbstoff für die qPCR genutzt wird, kann direkt im Anschluss zur qPCR eine Schmelzkurve aufgenommen werden. Diese gibt Auskunft, wie rein die Amplikons vorliegen. Für die Schmelzkurve wird direkt nach dem letzten Zyklus die Temperatur kontinuierlich erhöht (meist von 45°C bis 95°C). Dabei wird durchgehend das Fluoreszenzsignal gemessen. Durch diese langsame Erhöhung der Temperatur denaturiert immer mehr DNA. Ist die DNA denaturiert, kann SYBR Green nicht mehr binden und die Fluoreszenz nimmt ab. Die Ursache des Zeitpunktes der Denaturierung der DNA liegt hauptsächlich im GC-Gehalt und der Länge der DNA begründet. Dies führt dazu, dass jedes PCR Produkt seinen eigenen Schmelzpunkt besitzt. Da die Amplikons in großer Zahl in der Probe enthalten sind, nimmt bei der Schmelztemperatur dieser Amplikons die Fluoreszenz sprunghaft ab (siehe Figur 2). Zur Darstellung der Schmelzkurve wird die Fluoreszenz gegen Temperatur aufgetragen. Zur einfachen Analyse kann die erste Ableitung der Fluoreszenz gegen die Temperatur dargestellt werden. Hierbei werden die Schmelzpunkte sichtbar (vergleiche die Darstellungsarten in Figur 2). Idealerweise ist bei einer PCR nur ein Schmelzpunkt vorhanden. Sind mehrere vorhanden, kann dies diverse Gründe haben. Hierzu zählen z.B. Bildung von Primer-Dimeren oder eine unspezifische Bindung der Primer. Die Schmelzkurvenanalyse stellt eine wichtige Methode bei der Optimierung einer qPCR dar.

Die untere Grenze des erfindungsgemäßen Verfahrens für den Nachweis einer Nukleinsäure, vorzugsweise doppelsträngige DNA, liegt zwischen 10 fg - 0,08 fg/$\mu$l, also z.B. bei 10, 9, 8, 7, 6, 5, 4, 3, 2, 1, 0,5, 0,4, 0,25, 0,1, 0,09 oder 0,08 fg/$\mu$l. Die Quantifizierung erfolgt vorzugsweise absolut, d.h. als absolute Quantifizierung.

Eine Probe, die biologisches Material enthält, gilt vorteilhafterweise dann als negativ für eine Nukleinsäure, d.h. sie enthält keine nachweisbare Nukleinsäure, wenn der Cp-Wert der zu messenden Probe dem Cp-Wert der Standardkurve entspricht, bei dem mit dem erfindungsgemäßen Verfahren der Cp-Wert nicht den Cp-Wert des Hintergrundes übersteigt, also gleich dem Hintergrundwert oder sogar niedriger ist. Mit "Hintergrund" ist gemeint, dass eine zu vermessende Probe keine Nukleinsäure enthält. Dies kann man z.B. durch die Zugabe von Nukleasen, wie z.B. DNasen, RNasen oder Säure zu einer solchen Negativ-Probe erreichen.

Die Probe kann flüssig sein oder fest, wobei eine feste Probe vorzugsweise verflüssigt wird, indem sie z.B. in einem wässrigem Medium gelöst oder suspendiert wird. Die Probe kann jede Flüssigkeit oder Feststoff sein, die/der biologisches Material enthält. Flüssigkeiten können z.B. Körperflüssigkeit eines Säugers, Vogels, Fisches, Reptils, Insekts sein, aber auch das Zytosol oder die Zellwand oder Zellmembran von Säugerzellen, Bakterienzellen, Pilzzellen inkl. Hefezellen, Fischzellen, Vogelzellen, Reptilienzellen, Viren oder Insektenzellen. Körperflüssigkeiten umfassen z.B. Speichel, Sekrete, Urin, Blut, Serum, Plasma, Sperma, Liquor, Brustmilch, Tränenflüssigkeit, etc. Flüssigkeiten können ferner die Fermentationsbrühe oder der Überstand einer Kultur der vorgenannten Zellen sein, insbesondere die Fermentationsbrühe oder der Zellkulturüberstand an sich bekannter oder neu entwickelter Produktionssysteme für biologisch und/oder synthetisch biologisch hergestellte Arzneimittel (beispielsweise CHO-Zellen, E. coli Zellen, Bacillus subtilis Zellen; Insektenzellen, Hefezellen etc.). Vorzugsweise ist die Probe ein biopharmazeutisches oder biotechnologisches Produkt.

Das biologische Material der Probe, die einem erfindungsgemäßen Verfahren zum Nachweis einer Nukleinsäure in einer Probe zugeführt wird, stammt vorzugsweise aus Säugerzellen, Bakterienzellen oder Pilzzellen, kann aber auch aus Fischzellen, Vogelzellen, Reptilienzellen, Viren oder Insektenzellen stammen. Säugerzellen im Sinne der Erfindung umfassen vorzugsweise Zellen vom Mensch, Maus, Hamster, Ratte, Hase, Kamel, Lama, Hund, Katze, Pferd, Kuh oder Schwein.

Das biologische Material kann Nukleinsäuren wie z.B. DNA (beispielsweise genomische DNA, Plasmid DNA, DNA aus Organellen), RNA (beispielsweise mRNA, rRNA miRNA, siRNA, und/oder tRNA), Proteine, Kohlenhydrate und/oder Fette etc. enthalten. Nukleinsäuren des biologischen Materials, vorzugsweise DNA oder RNA wie unter anderem oben beschrieben (sowohl einzelsträngig - bisweilen "ss" abgekürzt, als auch doppelsträngig - bisweilen "ds" abgekürzt), werden im biologischen Material mit dem erfindungsgemäßen Verfahren nachgewiesen, wohingegen Proteine, Kohlenhydrate und/oder Fette vorzugsweise nicht nachgewiesen werden.

Das biologische Material ist vorzugsweise zur Verabreichung an Tieren oder Menschen vorgesehen. Die Verabreichung an den Menschen ist bevorzugt. Die intravenöse Verabreichung des biologischen Materials an Mensch oder Tier ist dabei besonders bevorzugt. Es ist von besonderem Interesse, dem Mensch keine Nukleinsäure zuzuführen, die in der Probe enthalten ist. Dies ist insbesondere bedeutsam, da biotechnologisch hergestellte Produkte für die Therapie, Diagnose, Kosmetik oder Lebensmittel oftmals aus Wirtszellen stammen, z.B. auch aus Säugerzellen, wie z.B. menschlichen Zellen/Zelllinien, und es wünschenswert ist, dass keine Nukleinsäuren dieser Wirtszellen bzw. Nukleinsäuren nur in Mengen innerhalb der von Behörden zugelassenen Grenzen dem Menschen (unvermeidlich) zugeführt werden. Das erfindungsgemäße Verfahren ermöglicht es nun, dass solche Nukleinsäure in einer Probe nachgewiesen werden können, um das biologische Material der Probe, das zur Verabreichung an Menschen vorgesehen ist, so sensitiv zu untersuchen, dass mögliche Gefahren oder unerwünschte Nebenwirkungen bei der Verabreichung weitestgehend vermieden oder sogar von vorneherein ausgeschlossen werden können. Dies liegt daran, dass das erfindungsgemäße Verfahren die bisherigen unteren Grenzen für den Gehalt an Nukleinsäuren in biologischen Material unterschreitet, so dass durch die höhere Sensitivität des erfindungsgemäßen Verfahren eine höhere Gewissheit an Sicherheit über die Menge an Nukleinsäure einer für die Produktion eingesetzten Wirtszelle im biologischen Material hergestellt werden kann.

Die im biologischen Material enthaltenen Nukleinsäuren können DNA oder RNA sein, doppelsträngig oder einzelsträngig. Die DNA kann genomische DNA, Plasmid-DNA, Cosmid-DNA, Bac-mid DNA, etc. sein, vorzugsweise ist die DNA genomische DNA. Im Fall des Nachweises von RNA wird vorteilhafter Weise eine reverse Transkription durchgeführt, um RNA in cDNA überzuführen. D.h. im Fall des Nachweises von RNA in einer Probe, die biologisches Material enthält, wird vorteilhafter Weise vor der Behandlung mit Protease eine reverse Transkription durchgeführt, schlussendlich aber wird RNA spätestens vor Schritt (d) in cDNA revers transkribiert, was im Umkehrschluss bedeutet, dass die reverse Transkription vor oder nach einem der Schritte (a) bis (c) durchgeführt werden kann. D.h. dass das erfindungsgemäße Verfahren zusätzlich zu den oben genannten Schritten (a) bis (d) auch den Schritt Unterziehen der Probe einer reversen Transkription umfassen kann. Bei der reversen Transkription werden entweder oligo-dT Primer oder zufällige 6-mer, 8-mer oder 10-mer Primer zusammen mit dem Enzym Reverse Transkriptase (RT) verwendet. Wenn die Nukleinsäure von Eukaryoten stammt, werden vorzugsweise oligo-dT Primer für die reverse Transkription verwendet. Nach der reversen Transkription wird das Enzym RT vorzugsweise inaktiviert.

Das biologische Material einer Probe enthält höchstens Nukleinsäure, vorzugsweise DNA. D.h. Ein erfindungsgemäßes Verfahren bezieht sich vorzugsweise auf den Nachweis einer Nukleinsäure in einer Probe, die biologisches Material enthält, wobei das biologische Material nicht mehr als 1 ng (=1000 pg), vorzugsweise nicht mehr als 500 pg, 400 pg, 300 pg, 200 pg, 100 pg, 50 pg, 25pg oder 10 pg pro Dosis enthält. Eine Dosis können 1, 5, 10, 20, 30, 40, 50, 60, 70, 80, 90, 100, 150, 200, 250 oder mehr Milliliter sein.

Die mit dem erfindungsgemäßen Verfahren nachzuweisenden Nukleinsäuren können von einer Wirtszelle stammen, die das biologische Material an sich darstellt oder aber das biologische Material herstellt. Im letztgenannten Fall stammen die im biologischen Material, das aus der Wirtszelle stammt (von dieser hergestellt wird), enthaltenen Nukleinsäuren letztendlich aus der Wirtszelle und sollen mit dem erfindungsgemäßen Verfahren gegebenenfalls nachgewiesen werden, sofern überhaupt nachweisbare Nukleinsäure in der Probe enthalten sind. Solche Wirtszellen sind hierin detaillierter beschrieben.

Wie eingangs ausgeführt, ist es eine bevorzugte Ausführungsform der vorliegenden Erfindung, dass biologisches Material, insbesondere biotechnologisch hergestelltes Material auf die mögliche Anwesenheit von Nukleinsäuren hin untersucht wird, die z.B. von Wirtszellen stammen, die bei der biotechnologischen Herstellung verwendet werden. Wirtszellen im Sinne der Erfindung können beispielsweise sein: Säugerzellen, Bakterienzellen, Pilzzellen, Fischzellen, Vogelzellen, Reptilienzellen, Insektenzellen oder Viren. Das biologische Material enthält Antikörper oder ein therapeutisches Protein, das insbesondere von einer Wirtszelle hergestellt wird/wurde.

Bevorzugte Säugerzellen im Sinne der Erfindung sind PER.C6, HEK-Zellen, Primatenzellen, z.B. Vero-Zellen, NS0-Zellen, CHO-Zellen, beispielsweise DUXB11, DG44, oder CHOK1, Maus-Hybridomzellen, Ratten-Hybridomzellen, oder Hasen-Hybridomzellen.

Der Ausdruck "Antikörper" umfasst jedweden Antikörper, Derivate oder funktionelle Fragmente davon, die immer noch ihre Bindungsspezifität aufweisen. Verfahren zum Herstellen von Antikörpern sind auf dem Gebiet hinlänglich bekannt und beschrieben, z. B. in Harlow und Lane "Antibodies, A Laboratory Manual", Cold Spring Harbor Laboratory Press, 1988, und Harlow und Lane "Using Antibodies: A Laboratory Manual" Cold Spring Harbor Laboratory Press, 1999. Der Ausdruck "Antikörper" umfasst auch Immunglobuline (Igs) unterschiedlicher Klassen (d. h. IgA, IgG, IgM, IgD und IgE) und Unterklassen (wie IgG1, IgG2 usw.) sowie daraus abgeleitete Moleküle. Diese Antikörper können beispielsweise für die Immunpräzipitation, Affinitätsreinigung und Immunlokalisation der Polypeptide oder Fusionsproteine der Erfindung sowie für das Überwachen des Vorhandenseins und der Menge solcher Polypeptide, z. B. in Kulturen rekombinanter Prokaryoten oder eukaryotischer Zellen oder Organismen verwendet werden. Die Definition des Ausdrucks "Antikörper" umfasst darüber hinaus Ausführungsformen wie chimäre, Einzelketten- und humanisierte sowie menschliche Antikörper sowie Antikörperfragmente wie unter anderem Fab-Fragmente. Antikörperfragmente oder -derivate umfassen darüber hinaus F(ab), F(ab)$_2$, F(ab')$_2$-, Fv-, scFv-Fragmente oder Antikörper mit einer einzelnen Domäne, z.B. Nanobodies oder Domain Antibodies, Antikörper mit einer einzelnen variablen Domäne oder eine einzelne variable Domäne von Immunglobulin, die nur eine variable Domäne umfasst, die VH oder VL sein kann, die ein Antigen oder Epitop unabhängig von anderen V-Regionen oder - Domänen spezifisch bindet; siehe z. B. Harlow und Lane (1988) und (1999), loc. cit. Solche einzelnen variablen Domänen von Immunglobulin umfassen nicht nur ein Polypeptid eines isolierten Antikörpers mit einer einzelnen variablen Domäne, sondern auch größere Polypeptide, die ein oder mehrere Monomere einer Polypeptidsequenz eines isolierten Antikörpers mit einer einzelnen variablen Domäne umfassen. Des Weiteren umfassen Antikörperfragmente oder -derivate bispezifische Antikörper, wie z.B. bispezifische Einzelketten-Antikörper (scFv), Diabodies, Tetrabodies oder DART-Antikörper. Verschiedene Verfahren sind auf dem Gebiet bekannt und können für die Herstellung solcher Antikörper und/oder Fragmente verwendet werden. Somit können die (Antikörper-) Derivate mithilfe von Peptidmimetika hergestellt werden. Darüber hinaus können Verfahren, die für die Herstellung von Einzelketten-Antikörpern beschrieben sind (siehe unter anderem das US-Patent 4, 946, 778) derart angepasst werden, dass sie Einzelketten-Antikörper herstellen, die für ein oder mehrere ausgewählte Polypeptide spezifisch sind. Darüber hinaus können transgene Tiere verwendet werden, um humanisierte Antikörper zu exprimieren, die für Polypeptide und Fusionsproteine dieser Erfindung spezifisch sind. Zur Herstellung von monoklonalen Antikörpern kann jedes beliebige Verfahren verwendet werden, das Antikörper bereitstellt, die durch kontinuierliche Zelllinienkulturen hergestellt werden. Beispiele für solche Verfahren umfassen das Hybridomverfahren (Köhler und Milstein, Nature 256 (1975, 494-497), das Triomverfahren, das Humane-B-Zellen-Hybridom-Verfahren (Kozbor, Immunology Today 4 (1983), 72) und das EBV-Hybridom-Verfahren zur Herstellung von humanen monoklonalen Antikörpern (Cole et al., Monoclonal Antibodies and Cancer Therapy, Alan R. Liss, Inc. (1985), 77-96). Eine Oberflächenplasmonresonanz, wie im BIAcore-System verwendet, kann herangezogen werden, um die Effizienz von Phagenantikörpern zu steigern, die an ein Epitop eines Zielpolypeptids binden, z. B. CD3 epsilon (Schier, Human Antibodies Hybridomas 7 (1996), 97-105; Malmborg, J. Immunol. Methods 183 (1995), 7-13). Es wird im Kontext dieser Erfindung darüber hinaus in Betracht gezogen, dass der Ausdruck "Antikörper" Antikörperkonstrukte umfasst, die in einem Wirt, wie hier nachstehend beschrieben, exprimiert werden können, z. B. Antikörperkonstrukte, die über unter anderem Viren oder Plasmidvektoren transfiziert und/oder transduziert werden können.

Ein Antikörper, der im erfindungsgemäßen biologischen Material enthalten ist, ist vorzugsweise ein Antikörper gegen EGFR, Her2, TA-MUC1, TF oder LeY ist.

Beispiele für bevorzugte Antikörper sind der anti-EGFR-Antikörper Cetu-GEX®, der anti-Her2 Antikörper TrasGEX®, der anti-TA-MUC1 Antikörper PankoMab-GEX® oder SeeloMab-GEX®, der anti-TF Antikörper GatoMab-GEX®, Karomab-GEX® oder Teltomab-GEX® oder der anti-LeY Antikörper LindoMab-GEX®.

Ein therapeutisches Protein, das im erfindungsgemäßen biologischen Material enthalten ist, ist vorzugsweise ein Hormon, Enzym oder Zytokin oder ein daraus abgeleitetes Protein oder Peptid.

Beispiele für ein bevorzugtes therapeutisches Protein ist das Hormon FSH, hCG, hLH, oder hGH. Weitere Beispiele für ein bevorzugtes therapeutisches Protein ist der Gerinnungsfaktor Faktor VII, Faktor FVIIa, Faktor FVIII, Faktor VIIIa, Faktor IX, Faktor IXa, Faktor X oder Faktor Xa ist oder Fusionsproteine und Derivate aus/mit diesen Proteinen. Wiederum weitere Beispiele für ein bevorzugtes therapeutisches Protein sind Enzyme, die Im Rahmen einer Enzymersatztherapie (EET) verwendet werden, wie z.B. Glukozerebrosidase oder Galaktosidase oder Fusionsproteine und Derivate aus/mit diesen Proteinen. Die EET ist ein therapeutisches Verfahren zur Behandlung von Enzymdefekten bei lysosomalen Speicherkrankheiten. Den Patienten werden dabei per Infusion oder Injektion rekombinante Enzyme zugeführt.

Die im Schritt (a) der erfindungsgemäßen Verfahren verwendete Protease kann prinzipiell jede Protease sein, vorzugsweise wird eine unspezifische Serinprotease, z.B. Proteinase K verwendet. Eine solche Protease hat ein sehr breites Erkennungsspektrum. Sie schneidet die Carboxylenden von aromatischen, hydrophilen und aliphatischen Aminosäuren). Dabei spaltet Proteinase K Proteine folgendermaßen: X - ↓ - Y -, wobei X eine aliphatische, aromatische oder hydrophobische Aminosäure und Y jedwede Aminosäure bedeutet. Proteinase K wird durch denaturierende Substanzen wie SDS aktiviert. Im erfindungsgemäßen Verfahren wird eine Protease, vorzugsweise Proteinase K genutzt, um Proteine, die im biologischen Material einer Probe vorhanden sind, zu entfernen. Eine Protease, die im erfindungsgemäßen Verfahren verwendet wird, ist vorteilhafterweise fei von kontaminierenden Nukleinsäuren, insbesondere frei von DNA.

Der Fachmann ist sich über die Inkubationszeiten einer Probe mit einer Protease im Klaren. Er wird die Probe vorzugsweise mindestens 1h oder länger, z.B. 2, 3, 4, 5, 6, 7, 8, 9, 10, 12, 18 oder 24h mit der Protease behandeln, z.B. über Nacht. Der Fachmann ist sich auch im Klaren über die Temperatur, bei der er die Probe mit Protease behandelt, vorzugsweise wird der Fachmann dazu eine Temperatur zwischen 37°C - 53°C verwenden, ggfs. kann der Fachmann SDS zum Spaltungsansatz geben. Genauso ist der Fachmann mit den einzusetzenden Mengen an Protease vertraut, er wird jedoch vorzugsweise zwischen 1-5 mg/ml einsetzen, z.B. 2 mg/ml. Für den Fall, dass die Protease inaktiviert werden soll, ist beispielsweise eine Inaktivierung bei 95°C für eine Dauer von zumindest 10 Minuten oder mehr vorgesehen.

Das Konzentrieren einer Probe im Schritt (b) des erfindungsgemäßen Verfahrens erfolgt vorzugsweise mittels Filtration unter Volumenreduktion.

Bei einer Filtration werden Substanzen, welche sich in einer Lösung befinden, abgetrennt. Hierbei stellt die Membran eine Barriere dar, welche für Substanzen aufgrund ihrer physikalischen oder chemischen Eigenschaften durchlässig ist oder diese aufhält. Membranen werden normalerweise anhand ihrer Porengröße klassifiziert. Eine bevorzugte Filtration, die beim erfindungsgemäßen Verfahren angewendet wird, ist die Ultrafiltration. Bei der Ultrafiltration beträgt die Porengröße durchschnittlich 0,001 - 0,1 $\mu$m. Die Membranen halten Moleküle mit einem Molekulargewicht von 300 bis 10.000.000 Da (Dalton) zurück. Vorzugsweise werden erfindungsgemäß Membranen mit einem 10k Filter benutzt, beispielsweise Amicon Ultrafiltrationseinheiten mit 10K Filter. Dies bedeutet, dass diese ein NMWL (Nominal Molecular Weight Limit) von 10.000 Da haben. Moleküle, deren Molekulargewicht kleiner als 10 kDa (Kilodalton) ist, können die Membran passieren. Schwerere Moleküle können dies nicht.

Durch Zentrifugation wird die Probe durch die fast vertikal ausgerichtete, aus regenerierter Zellulose bestehende Membran gedrückt und von mehreren Milliliter auf wenige Mikroliter aufkonzentriert. Vorzugsweise wird eine Probe im Schritt (b) des erfindungsgemäßen Verfahrens um den Faktor 10-20 aufkonzentriert.

Eine bevorzugte Vorrichtung zum Durchführen von Schritt (b) des erfindungsgemäßen Verfahrens ist eine Amicon Ultra-4 Zentrifugen-Filtereinheit (10 K).

Das Anreichern der Nukleinsäure in Schritt (c) des erfindungsgemäßen Verfahrens erfolgt vorzugsweise mittels Affinitätschromatographie oder Fällung. Vorzugsweise erfolgt die Affinitätschromatographie durch Silika-Adsorption oder Adsorption an polymere Partikel. Die Silika-Adsorption erfolgt vorzugsweise mittels einer Silika-basierten Membran.

Ein gängiges Prinzip beim Anreichern von Nukleinsäure basiert auf Affinitätschromatographie, insbesondere an eine Silikamembran. Eine Silikamembran besteht aus Quarz (SiO2), welches an OH- - Gruppen gebunden ist. Eine Nukleinsäure ist z.B. in Wasser gelöst. Sowohl diese, als auch die Silikamembran, sind mit einer Hydrathülle umgeben. Chaotrope Salze bestehen aus Ionen, welche hydrophobe Effekte vermindern. Hierzu gehören z.B. SCN-, H2PO4-; NH4+, K+ oder Guanidin. Durch Zugabe von chaotropen Ionen wird die Hydrathülle der Membran und der DNA destabilisiert, und die DNA bindet an die Silikamembran. Man nimmt an, dass sich intermolekulare Wasserstoffbrückenbindungen zwischen dem Rückgrat der DNA und der OH--Gruppe der Membran ausbilden. Durch diese Bindungen wird die DNA an die Membran adsorbiert. Eine andere Annahme beschreibt eine Sättigung der negativen OH-Gruppen mit positiv geladenen Ionen. Diese positiven Ionen bilden eine Kationenbrücke zum Rückgrat der DNA aus. Hierdurch wird die Nukleinsäure an die Silikamembran fest gebunden und kann gewaschen werden. Als Wasch/Elutionspuffer sollten vorzugsweise Lösungen mit hohen Konzentrationen an chaotropen Salzen oder Ethanol gewählt werden.

Zu beachten ist, dass im Schritt (c) des erfindungsgemäßen Verfahrens vorzugsweise das gleiche Prozedere durchgeführt wird als ob eine Nukleinsäure isoliert werden wollte. Das heißt, es wird nicht von vorne herein davon ausgegangen, dass eine Nukleinsäure frei, also in Lösung, in der Probe, die biologisches Material enthält, vorliegt. So werden z.B. die Vorschriften eines kommerziellen Kits, wie unten beschrieben, beachtet.

Bevorzugte Beispiele für polymere Partikel sind Polystyren-Partikel, wie z.B. Dynabeads.

Eine Fällung von Nukleinsäuren, insbesondere DNA wird nach an sich bekannten Verfahren durchgeführt.

Die Erfinder stellten fest, dass die Ausbeute an Nukleinsäuren, insbesondere DNA bei der Elution bzw. dem Abwaschen mit Wasch/Elutionspuffer von der Silikamembran bei 45°C besser war als bei Raumtemperatur (19-26°C). Daher ist es eine bevorzugte Ausführungsform des erfindungsgemäßen Verfahrens, im Schritt (c) das Eluieren der Nukleinsäure, insbesondere DNA von der Silika-basierten Membran bei 45°C durchzuführen.

Beispiele für Vorrichtungen zum Anreichern der Nukleinsäure in Schritt (c) des erfindungsgemäßen Verfahren sind das NucleoSpin® Plasma XS Kit, QIAamp UCP Pathogen Mini Kit, QIAamp DNA Investigator Kit, QIAamp Viral RNA Mini Kit, NucleoSpin® Tissue XS Kit, NucleoSpin® Trace Kit, nexttec clean Column, DNA Extraction EZ-Kit, forensicGEM Tissue Kit, wobei das QIAamp DNA Investigator Kit bevorzugt ist.

Das Nachweisen der Nukleinsäure mittels quantitativer PCR (qPCR) im Schritt (d) des erfindungsgemäßen Verfahrens stellt vorzugsweise auf solche Nukleinsäuren ab, die der Fachmann in der Probe erwarten würde, die biologisches Material enthält. Zum Beispiel würde er im Fall einer Probe, die biologisches Material enthält, das mittels einer Wirtszelle biotechnologisch hergestellt wurde, auf solche Nukleinsäuren abstellen, die von der Wirtszelle möglicherweise in der Probe enthalten sind. Das heißt, dass in einer bevorzugten Ausführungsform im Schritt (d) des erfindungsgemäßen Verfahren auf Nukleinsäuren, insbesondere auf DNA der Wirtszelle abgestellt wird, die für die biotechnologische Herstellung des biologischen Materials verwendet wurde. Beispielsweise würde im Fall der Verwendung von E. coli auf E. coli DNA abgestellt werden, im Fall von Hefe auf Hefe DNA, im Fall von CHO Zellen auf CHO DNA oder im Fall einer menschlichen Zelle auf menschliche DNA. Selbstredend weiß der Fachmann, dass er bei Verwendung einer der hierin beschriebenen Wirtszellen für die Herstellung von biologischem Material im Schritt (d) auf Nukleinsäuren, insbesondere DNA der hierin beschriebenen Wirtszellen abstellt. Im Fall von menschlicher DNA wird vorzugsweise auf repetitive Elemente abgestellt, vorzugsweise auf repetitive Elemente in Säuger-DNA. Repetitive Elemente können vorzugsweise Alu-Sequenzen oder Alu-äquivalente Sequenzen sein.

Der beim Nachweisen der Nukleinsäure nötige Standard ist ein solcher wie oben stehend definiert. Demzufolge bezieht sich Schritt (d) vorzugsweise auf Nachweisen der Nukleinsäure mittels quantitativer PCR, wobei die quantitative PCR auf Nukleinsäuren abstellt, die der Fachmann in der Probe erwarten würde, die biologisches Material enthält, wie oben stehend näher beschrieben.

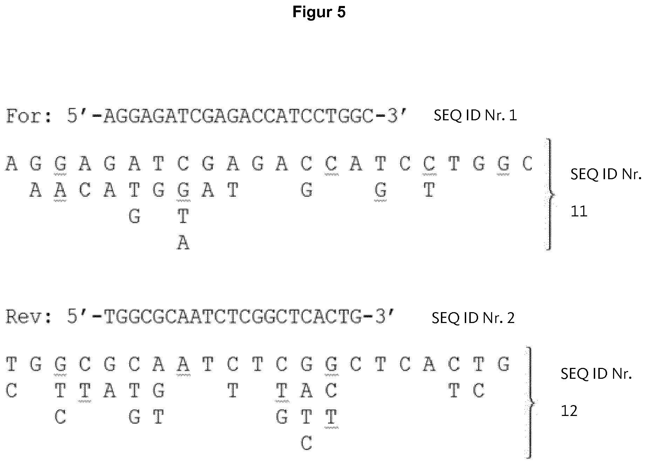

Im Schritt (d) des erfindungsgemäßen Verfahren wird vorzugsweise das Primerpaar hat die SEQ ID Nr. 1 und SEQ ID Nr. 2. Ein anderes besonders bevorzugtes Primerpaar hat die SEQ ID Nr. 3 und SEQ ID Nr. 4. Andere bevorzugte Primerpaare sind die mit der SEQ ID Nr. 5 und 6. Weitere bevorzugte Primerpaare sind diejenigen mit den SEQ ID Nr. 1 und 7, 1 und 8, 1 und 9, 1 und 10, oder 11 und 12 (siehe auch Figur 5). Ebenso bevorzugt ist ein Gemisch aus den Primerpaaaren mit den SEQ ID Nr. 1 und 7-10.

Eukaryoten tragen verschiedene Typen mobiler DNA-Elemente in ihren Genomen, die in unterschiedlich großer Zahl auftreten und aufgrund von Sequenzhomologien klassifiziert werden können. Das menschliche Genom besteht zu mehr als 45% aus mobilen Elementen. Eine der größten Gruppen mobiler Elemente bilden die Alu-Sequenzen. Diese sind in hoher Kopienzahl, also repetitiv, und gleichmäßig über das menschliche Genom verteilt. Innerhalb der Genome gibt es verschiedene Arten von repetitiven Sequenzen. Die Kopien können in großer Zahl direkt aneinander gereiht als "Tandem Repeats" vorliegen, wie z.B. im Telomerbereich von Chromosomen. Alu-Elemente gehören zu den sogenannten SINEs (short interspersed elements). SINEs sind über das ganze Genom verteilt und haben eine maximale Größe von 500 bp (Basenpaare). Alu-Elemente sind kleine, 300 bp große Sequenzen, welche vielfach (mehr als eine Millionen Kopien) im Genom wiederholt werden. Alu-Sequenzen repräsentieren somit nicht nur die größte Menge der mobilen Elemente sondern auch der SINEs. Aufgrund dieser hohen Anzahl an Kopien stellen die Alu-Sequenzen mehr als 10% der menschlichen Genommasse.

In der Publikation "Human DNA quantitation using Alu element - based polymerase chain reaction" von J. A. Walker et al (2003), Anal. Biochem. 315, 122-128 wird eine qPCR basierend auf der Yb8 Alu-Subfamilie beschrieben. Durch die human-genomische Spezifität der Yb8 Alu- Elemente und der hohen Kopienzahl von 1852, welche im ganzen Genom verteilt sind, eignen sich diese Alu-Sequenzen besonders gut zur spezifischen Quantifizierung von menschlicher DNA. Der Einsatz von Primerpaaren, die auf die Yb8 Alu-Subfamilie abstellen ist eine bevorzugte Ausführungsform im Sinne der vorliegenden Erfindung. Ein besonders bevorzugtes Primerpaar hat die SEQ ID Nr. 1 und SEQ ID Nr. 2. Ein anderes besonders bevorzugtes Primerpaar hat die SEQ ID Nr. 3 und SEQ ID Nr. 4. Andere bevorzugte Primerpaare sind die mit der SEQ ID Nr. 5 und 6. Weitere bevorzugte Primerpaare sind diejenigen mit den SEQ ID Nr. 1 und 7, 1 und 8, 1 und 9, 1 und 10, oder 11 und 12. Ebenso bevorzugt ist ein Gemisch aus den Primerpaaren mit den SEQ ID Nr. 1 und 7-10.

Repetitive Sequenzen sind nicht ausschließlich in menschlicher DNA zu finden, sondern auch in anderen Genomen. In CHO-Zellen (Chinese Hamster Ovary) wurden mehrere repetitive Elemente gefunden, welche eine signifikante Homologie zu den humanen Alu-Sequenzen aufweisen, weshalb diese CHO Alu-äquivalente Elemente genannt werden. Aufgrund ihrer spezifischen Sequenz und ihrer hohen Kopienzahl eignen sich diese CHO Alu-äquivalenten Sequenzen gut als Primer zur Quantifizierung von CHO-DNA. Dementsprechend Der Einsatz von Primerpaaren, die auf CHO Alu-äquivalente Elemente abstellen ist eine bevorzugte Ausführungsform im Sinne der vorliegenden Erfindung. Ein besonders bevorzugtes Primerpaar hat die Sequenz 5'-TGGAGAGATGGCTCGAGGTT-3' (SEQ ID Nr. 5) bzw. 5'-TGGTTGCTGGGAATTGAACTC-3' (SEQ ID Nr. 6).

Ferner betrifft die vorliegende Erfindung ein Kit zum Durchführen eines erfindungsgemäßen Verfahrens, umfassend

(a) Protease, vorzugsweise Proteinase K;

(b) Mittel zum Konzentrieren von flüssigen Proben, vorzugsweise eine Vorrichtung für eine Ultrafiltration unter Volumenreduzierung;

(c) Mittel für die Affinitätschromatographie, vorzugsweise eine Vorrichtung, die von Silika-Adsorption Verwendung macht; und

(d) DNA-Polymerase und ein Primerpaar mit den SEQ ID Nr. 1 und 2, SEQ ID Nr. 3 und SEQ ID Nr. 4, SEQ ID Nr. 5 und 6, SEQ ID Nr. 1 und 7, SEQ ID Nr.1 und 8, SEQ ID Nr.1 und 9, SEQ ID Nr.1 und 10, SEQ ID Nr. 11 und 12 oder ein Gemisch aus den Primerpaaren mit den SEQ ID Nr. 1 und 7-10 zum Amplifizieren von repetitiven Elementen in DNA, vorzugsweise menschlicher DNA.

Ausführungsformen, die im Zusammenhang mit den erfindungsgemäßen Verfahren beschrieben sind, treffen auch auf die Bestandteile (a) bis (d) des erfindungsgemäßen Kits zu, *mutatis mutandis.*

## Figuren

**Figur 1:** Prinzip der absoluten Quantifizierung von DNA mittels qPCR

**Figur 2:** Schmelzkurve und Schmelzpunkte (Erläuterungen siehe Text) einer spezifischen und einer unspezifischen qPCR. Abb. nach Roche 2008.[6] Die grüne Kurve ist eine spezifische qPCR, welche daran erkennbar ist, dass nur ein steiler Abfall der Schmelzkurve und somit nur ein Schmelzpunkt vorhanden ist. Die blaue Kurve ist hingegen unspezifisch, was an zwei Maxima erkennbar ist.

**Figur 3:** Standardgerade der E1-DNA inklusive Standardabweichung Die dazugehörigen Messwerte sind Tabelle 11 zu entnehmen; rote Messpunkte sind Messwerte der E1-DNA, blaue Messpunkte die der Negativkontrolle (blauer Messpunkt = Schnittpunkt mit dem Nullpunkt der y-Achse).

**Figur 4:** Am LightCycler480 aufgenommene und abgespeicherte Standardgerade zur Bestimmung humaner DNA Konzentrationen.

**Figur 5:** Alu-Primersequenzen der SEQ ID Nr. 1 und 11 sowie 2 und 12. Gezeigt sind mögliche Permutationen des Forward-Primers (obere Sequenz) und des Reverse-Primers (untere Sequenz), die in SEQ ID Nr. 11 bzw. 12 reflektiert sind.

## Beispiele

## Beispiel 1

### 1. Durchführung der human qPCR mit Yb8-Primern

Zunächst wurde der Mastermix für alle Proben hergestellt. Die Endkonzentration des Forward Primer (SEQ ID Nr. 3) beträgt 0,15 μM, die des Reverse Primer (SEQ ID Nr. 4) 0,2 μM. Durch mehrmaliges Klopfen an das Röhrchen erfolgte eine Durchmischung des PCR-Mastermixes. Zur Durchführung der Negativkontrollen wurden in fünf Vertiefungen jeweils 20 μl PCR-Mastermix pipettiert und diese direkt mit einem PCR-Deckel verschlossen. Mit Hilfe der Multipette Xstream wurden in jede benötigte Vertiefung der MWP (Mehrloch-platte) 17 μl PCR-Mastermix vorgelegt. Danach wurden 3 μl Probe hinzugegeben. Mit Hilfe des Schabers wurde die MWP mit Folie gut verschlossen, bei 1500 rpm für 2 Minuten abzentrifugiert und direkt in den LightCycler gelegt. Anschließend wurde der PCR-Lauf mit folgenden Bedingungen gestartet:

| Schritt | Temperatur | Zeit | Temperaturab-/ anstieg |
|---|---|---|---|
| Denaturation | 95°C | 5 s | 4,4°C/s |
| Annealing + Elongation | 71°C | 20 s | 4,4°C/s |

### 2. Probenvorbereitung von CetuGEX™ zur späteren Quantifizierung der humanen Rest-DNA mittels qPCR

Wie eingangs beschrieben, darf, wie von z.B. der FDA vorgeschrieben, eine Rest-DNA Menge von 100 pg/Dosis der Wirtszelle nicht überschritten werden. Da bei CetuGEX™ eine Dosis in 200 ml vorgesehen ist, darf die DNA-Menge der den Antikörper herstellenden Wirtszelle nicht mehr als 0,5 pg/ml betragen. Diese niedrige DNA-Konzentration war mit Hilfe der humanen qPCR alleine nicht zu messen. Daher fanden die Erfinder einen Weg, dieses Problem zu beheben und stellten die erfindungsgemäßen Verfahren bereit.

Die Proben wurden in 3 Schritten vorbereitet: Als erstes erfolgte ein Proteinase K-Verdau, bei welchem die Proteine, die sich in großer Menge in CetuGEX™ befinden, entfernt wurden, um den folgenden Schritt vorzubereiten. Im zweiten Schritt wurde mit Hilfe der Ultrafiltration die DNA aufkonzentriert. Diese Lösung wäre ohne die Vorbehandlung mit Proteinase K zu viskos gewesen. Im letzten Schritt wurden die aufkonzentrierten Proben aufgereinigt, da diese möglicherweise PCR inhibierende Substanzen enthielten. Diese Aufreinigung wurde mit Hilfe eines kommerziell erhältlichen Kits durchgeführt.

2.1 Proteinase K-Verdau von CetuGEX™

4 ml Probe wurden mit 40 U Proteinase K und 40 µl 10% SDS-Lösung versetzt und gut vermengt. Über Nacht wurde diese Mischung bei 53°C inkubiert. Dabei wurde das 15 ml-Röhrchen gut mit Parafilm verschlossen, um Kontaminationen zu vermeiden.

2.2 Ultrafiltration von CetuGEX™ zur Aufkonzentrierung humaner Rest-DNA

Nach dem Verdau mit Proteinase K wurde die Probe 2 min abzentrifugiert (4000 rcf) und in eine Amicon Ultrafiltrations Einheit gegeben. Diese wurde im nächsten Schritt für 8 Min bei 4000 rcf zentrifugiert (Hettich-Zentrifuge). Der Überstand im Filter wurde entnommen, in 2 ml-Röhrchen umgefüllt, 5 Min bei 12.300 rcf in der Tischzentrifuge abzentrifugiert und das Volumen mit Hilfe der Multipette Xstream bestimmt. Dieses Volumen bildete die Grundlage für die Berechnung des Wasservolumens für den folgenden Waschschritt. Das Präparationsvolumen auf dieser Stufe sollte insgesamt 400 µl pro Probe ergeben, also Volumen des Ultrafiltrats zusammen mit Waschvolumen. Gewaschen wurde dementsprechend mit dem Differenzvolumen von 400 µl abzüglich des Ultrafiltratvolumens. Der Waschschritt wurde mit Wasser, PCR Grade, durchgeführt. Jede Seite des Filters wurde 7-mal gewaschen und dieses Waschvolumen zum Ultrafiltrat gegeben.

2.3 Aufreinigung der aufkonzentrierten CetuGEX™-Proben für die Messung humaner DNA mittels qPCR

In den folgenden Punkten werden verschiedene DNA-Aufreinigungsmethoden, die auf den jeweils im Titel genannten Kits beruhen, beschrieben. Wo nicht anders angegeben, handelte es sich um Präparationen im kleinen Maßstab, die in einer Tischzentrifuge (VWR) durchgeführt werden konnten.

2.3.1 NucleoSpin® Plasma XS Kit

Nachdem die Probe mit Proteinase K-Verdau behandelt und ultrafiltriert worden war, wurden 400 µl der Probe mit 600 µl BB Puffer versetzt. Das Röhrchen wurde drei Mal invertiert, für 3 s gevortext und kurz ab-zentrifugiert. Die folgenden Präparationsschritte konnten an der VWR-Tischzentrifuge durchgeführt werden. Zwei Mal wurden je 500 µl der Mischung auf die im Kit enthaltene Säule gegeben, bei 2.000 x g für 30 s zentrifugiert und der Auffangbehälter verworfen. Nach einem weiteren Zentrifugationsschritt bei 11.000 x g für 5 s wurde 500 µl Wasch-puffer (WB) auf die Säule gegeben. Diese wurde dann bei 11.000 x g für 30 s zentrifu-giert, ein weiteres Mal mit 250 µl WB gewaschen und für 3 min bei 11.000 x g zentrifu-giert. Die Säule wurde in ein DNA-freies Eppendorf Röhrchen überführt, 40 µl Elutions-puffer zugegeben und bei 11.000 x g für 30 s zentrifugiert.

2.3.2 QIAamp DNA Investigator Kit

Nachdem die Probe mit Proteinase K behandelt und ultrafiltriert worden war, wurden 400 µl der behandelten Probe mit 40 µl AW1 Puffer und 1 ml AW2 Puffer versetzt. Die Mischung wurde 10 s mit einem Vortexer gemischt und 720 µl davon auf eine QIAamp MinElute Säule gegeben. Nach der Zentrifugation bei 6.000 x g für 1 min wurde der Auffangbehälter gewechselt und die restliche Probe aufgegeben. Nach erneuter Zentrifugation unter den gleichen Bedingungen und Wechseln des Auffangbehälters wurden 500 µl des AW2 Puffers auf die Säule gegeben und bei 6.300 x g für 1 min zentrifugiert. Nach abermaligen Wechseln des Auffangbehälters wurde durch Zentrifugation bei maximaler Geschwindigkeit (12.300 x g) für 3 min die membranartige, DNA-bindende Matrix von Flüssigkeitsüberschüssen befreit. Die Säule wurde zur Elution in ein DNA-freies Eppendorf Röhrchen gegeben, 40 µl ATE Puffer auf die Mitte der Membran pipettiert und für 10 min bei Raumtemperatur inkubiert. Nach der Zentrifugation bei 12.300 x g für 1 min befanden sich durch-schnittlich etwa 38 µl eluierte DNA im Eppendorf-Röhrchen.

2.3.3 QIAamp UCP Pathogen Mini Kit

400 µl Probe und 200 µl APL2 wurden für 30 s gemischt. Nach Zugabe von 300 µl Ethanol wurde erneut für 30 s gevortext. 600 µl der Mischung wurden auf das QIAamp UCP Mini Spin column gegeben und bei 6.000 x g für 30 s zentrifugiert (VWR-Tischzentrifuge). Nach Auswechseln des Auffangbehälters wurde der Rest der Mischung auf die Säule pipettiert und wiederum bei den gleichen Bedingungen zentrifugiert. Ein weiteres Mal wurde der Auffangbehälter gewechselt und 600 µl APW1 Puffer auf die Säule gegeben. Nach der Zentrifugation bei 6.000 x g für 1 min wurde der Auffangbehälter gewechselt. Nach der Zugabe von 750 µl APW2 Puffer auf die Säule wurde diese bei 13.200 x g für 3 min zentrifugiert. Zur Entfeuchtung der Membran wurde die Säule in einen neuen Auffangbehälter gegeben, bei 12.300 x g für 1 min zentrifugiert und danach die Säule bei offenem Deckel im Heizblock, bei 56°C, für 3 min in-kubiert. Die Säule wurde nun in einem DNA-freien Eppendorf Röhrchen platziert, und nach Zugabe von 40 µl AVE Puffer, Inkubation für 1 min und Zentrifugation bei 12.300 x g befand sich die eluierte DNA im Röhrchen.

2.3.4 NucleoSpin® Tissue XS

Nachdem die Probe mit Proteinase K Verdau behandelt und ultrafiltriert worden war, wurden 400 µl der Probe mit 400 µl B3 Puffer und genauso viel Ethanol versetzt. Diese Mischung wurde gemischt und 400 µl davon auf die mitgelieferte Säule gegeben. Bei 11.000 x g wurde die Säule für eine Minute zentrifugiert, der Auffangbehälter gewechselt und in zwei weiteren Schritten die restliche Mischung auf die Säule aufgetragen und zentrifugiert. Nachdem der Auffang-behälter abermals gewechselt wurde, ist zum Waschen ein Volumen von 50 µl B5 Puffer auf die Säule gegeben worden; eine Zentrifugation bei 11.000 x g für eine Minute schloss sich an. Der Durchfluss wurde verworfen und die Säule mit weiteren 50 µl des B5 Puffers ein zweites Mal gewaschen. Abermals wurde die Säule bei 11.000 x g zentrifugiert. In ein DNA-freies Eppendorf-Röhrchen wurde die Säule hineingegeben und 40 µl BE Puffer auf die Säule gegeben. Die eluierte DNA wurde durch Zentrifugieren bei 11.000 x g für eine Minute in das Eppendorf-Röhrchen überführt.

2.3.5 NucleoSpin® Trace Kit

Wegen der großen Volumina musste diese Methode in der Hettichzentrifuge durchgeführt werden. Abweichend von den vorherigen DNA-Präparationsmethoden musste hier keine Ultrafiltration vorgeschaltet werden. Nachdem 4 ml Probe mit Proteinase K-Verdau behandelt worden waren, wurde dieses Volumen mit 8 ml FLB und 3,5 ml Ethanol versetzt. Das Gemisch wurde gut gemischt und auf die Säule des Kits gegeben. Nach Zentrifugation bei 3.000 x g für 3 min wurde der Auffangbehälter durch einen neuen Behälter ersetzt und die Säule drei Mal gewaschen. Die Waschschritte erfolgten einmal mit 2,5 ml BW und zwei Mal mit 5 ml B5 Puffer. Nach Zugabe der jeweiligen Waschpuffer wurde jedes Mal für 3 min bei 3.000 x g zentrifugiert. Nach abermaligem Wechseln des Auffangbehälters wurde durch 10 minütiges Zentrifugieren bei 3.000 x g die Silikamembran von überschüssiger Flüssigkeit befreit. Nachdem an die Säule das, im Kit enthaltene, 1,5 ml-Röhrchen angesetzt und beides zusammen in einem 50 ml-Röhrchen zur Zentrifugation gegeben worden war, wurden 100 µl BE Puffer auf die Silikamembran gegeben, um die Elution einzuleiten. Nach zweiminütiger Einwirkzeit bei Raumtemperatur wurde die Konstruktion bei 3.000 x für 3 min zentrifugiert. Die eluierte DNA befand sich im 1,5 ml-Röhrchen.

2.3.6 Nexttec clean Columns

Bevor die Nexttec clean Columns genutzt werden können, müssen diese äquilibriert werden. Dazu wurden 350 µl Präparationslösung, also die vorher verwendete Elutions-lösung für DNA auf die Säulen gegeben und diese für mindestens 5 min bei Raumtemperatur inkubiert. Anschließend wurde die Lösung für 1 min bei 350 x g abzentrifugiert. Die Säule wurde in ein DNA-freies Eppendorf Tube gegeben. Nun wurden 100 µl Probe auf die Säule gegeben, für 3 min bei Raumtemperatur inkubiert und bei 700 x g für 1 min zentrifugiert. Diese Methode stellt eine Gelfiltrationsmethode dar, basiert also nicht auf einer DNA-bindenden Matrix.

2.3.7 DNA Extraction EZ-Kit

Nachdem die Probe mit Proteinase K Verdau behandelt und ultrafiltriert worden war, wurden 500 µl der Probe mit 20 µl De-tergent Combo-Lösung versetzt und kurz gemischt. Diese Mischung wurde für 10 min bei 60°C inkubiert. 1 µl Glykogen wurde mit 500 µl Natriumiodid vermischt und diese Mischung zu der Probenmixtur gegeben. Nach erneutem Vortexen wurde ein weiteres Mal bei 60°C für 10 min inkubiert. Danach wurden zur DNA-Fällung 900 µl Isopropanol zu der Mischung zugegeben, gevortext und bei Raumtemperatur für 30 min inkubiert. Die Mischung wurde bei 12.000 rpm für 10 min zentrifugiert und der Überstand vorsich-tig abgegossen. 1,8 ml Waschpuffer wurden zu dem DNA-Pellet zugegeben; das Röhr-chen wurde gevortext. Zur Pelletierung wurde weitere 10 min bei 12.000 rpm zentrifugiert. Der Überstand wurde vorsichtig abgegossen und das Pellet für 1 h an der Luft getrocknet. Mit 50 µl Wasser, PCR Grade, wurde das Pellet resuspendiert.

2.3.8 forensicGEM Tissue Kit

4 ml Probe wurden unverdaut in eine Amicon Ultafiltrationseinheit gegeben und mittels Zentrifugation bei 4.000 rpm in der Hettich-Zentrifuge bis auf ca. 50 µl aufkonzentriert (Dauer ca. 50 min). Das Volumen der Probe wurde bestimmt und jede Membran der Ultrafiltrationseinheit 7 x mit so viel Wasser gewaschen, dass das Gesamtvolumen von Waschwasser und Probe 89 µl ergab. Ein Mischvorgang und eine kurze Zentrifugation schlossen sich an. Nach Zugabe von 10 µl 10xPuffer und 1 µl prepGEM wurde die Mischung erst für 15 min bei 75°C und direkt danach für 5 min bei 95°C inkubiert. Diese Lösung konnte direkt für qPCR verwendet werden (siehe 3.4.1).

2.3.9 Testen der humane DNA- freien Kits mit CetuGEX™

Nachdem nachgewiesen worden war, dass vier der Kits kontaminationsfrei waren, wurden diese mit CetuGEX™ getestet und miteinander verglichen. In Tabelle 1 ist erkennbar, dass mit Hilfe der Aufreinigung des NucleoSpin® Tissue Kits und des QIAamp DNA Investigator Kits die niedrigsten Cp-Werte erreichbar waren.

**Tabelle 1:** Cp-Mittelwerte der qPCR von CetuGEX mit Aufreinigung durch verschiedene Kits.

|  | CetuGEX™ | CetuGEX™ + 2 pg humane DNA |
|---|---|---|
| **forensicGEM Tissue Kit** | – | – |
| **NucleoSpin® Trace Kit** | 38,23 | 36,12 |
| **NucleoSpin® Tissue XS Kit** | 34,44 | 31,35 |
| **QIAamp DNA Investigator Kit** | 34,63 | 32,15 |

2.3.9.1 Testen des QIAamp DNA Investigator Kits und des NucleoSpin® Tissue Kits mit CetuGEX™ und verschiedenen DNA Standard Konzentrationen

Nach Eingrenzung der Kits auf zwei Stück mit den besten Vorergebnissen wurde ein letzter Versuch zum direkten Vergleich des NucleoSpin® Tissue XS Kit und des QIAamp DNA Investigator Kit durchgeführt. Als Probe wurde CetuGEX™, teilweise mit Standards versetzt, genutzt. Hierbei wurden mit jeder Probe drei Säulen getestet und jede Säule in Dreifachbestimmung vermessen. In **Fehler! Verweisquelle konnte nicht gefunden werden.** und **Fehler! Verweisquelle konnte nicht gefunden werden.** sind die einzelnen Cp Werte einzusehen. Tabelle 2 zeigt die Mittelwerte der einzelnen Säulen und die Mittelwerte der drei Säulen einer Probe.

**Tabelle 2:** Cp Mittelwerte des Vergleichstest von NucleoSpin® Tissue XS Kit und QIAamp DNA Investigator Kit. Vermessen wurden CetuGEX™-Ansätze ohne Zugabe von Standard, mit Zugabe von 2 pg, 20 pg und 200 pg DNA pro Säule; „Differenz": siehe Text.

| Kit | Zugabe humane DNA | - | 2 pg | 20 pg | 200 pg |
|---|---|---|---|---|---|
| **NucleoSpin® Tissue XS Kit** | Ø Cp Probe 1 | 32,47 | 30,93 | 27,23 | 24,54 |
|  | Ø Cp Probe 2 | 35,05 | 34,73 | 31,78 | 28,53 |
|  | Ø Cp Probe 3 | 35,81 | 34,87 | 33,48 | 28,15 |
|  | Ø Gesamt | 34,44 | 33,51 | 30,83 | 27,07 |
| **QIAamp DNA Investigator Kit** | Ø Cp Probe 1 | 31,19 | 29,64 | 27,09 | 23,72 |
|  | Ø Cp Probe 2 | 36,17 | 32,94 | 32,09 | 27,82 |
|  | Ø Cp Probe 3 | 34,40 | 32,36 | 31,61 | 29,45 |
|  | Ø Gesamt | 33,92 | 31,65 | 30,26 | 27,00 |
| **Differenz** |  | 0,52 | 2,18 | 0,56 | 0,07 |

Es ist ersichtlich, dass im Gesamtvergleich das QIAamp DNA Investigator Kit niedrigere Cp-Werte aufweist. Deutlicher wird dies noch in der Spalte "Differenz". Dort wurde Folgendes berechnet:
Ø Gesamt (NucleoSpin® Tissue XS Kit) - Ø Gesamt (QIAamp DNA Investigator Kit)

Die positiven Werte in der Spalte "Differenz" besagen, dass das QIAamp DNA Investigator Kit im Mittelwert aller Cp-Werte einer Probe (z.B. CetuGEX™ + 2pg) durchgehend niedrigere Werte erbrachte als das Vergleichskit. Bei einem negativen Wert hätte das NucleoSpin Tissue XS Kit einen niedrigeren Gesamt-Cp-Wert gehabt. Es wurde entschieden, mit dem QIAamp DNA Investigator Kit weiterzuarbeiten.

3. Verbesserung der Elution der DNA bei dem QIAamp DNA Investigator Kit

Da sich das QIAamp DNA Investigation Kit als das Geeignetste zur Aufreinigung von CetuG-EX™ erwiesen hatte, wurde dieses für die DNA Präparation weiter verwendet. Da der Cp-Wert von CetuGEX™ nach Aufreinigung teilweise noch sehr hoch war, sollte der DNA Gehalt erhöht werden. Dazu wurden Versuche zur Verbesserung der Elution der DNA im QIAamp DNA Investigator Kit durchgeführt. Alle Experimente wurden mit 4 ml CetuGEX™ durchgeführt. Vor der Aufreinigung wurde ein Proteinase K-Verdau und eine Ultrafiltration durchgeführt. Danach erfolgte eine Aufreinigung über das QIAamp DNA Investigator Kit. Nur die Elution wurde in den einzelnen Versuchen verändert. Die Auswertung erfolgte mittels humaner qPCR.

Es wurde vermutet, dass sich die DNA evtl. besser bei 45°C löst als bei Raumtemperatur und dadurch mehr DNA eluiert wird. Deswegen wurde bei 2 Proben die Einwirkung des Puffers bei 45° getestet. Die beiden Vergleichsproben wurden, wie im Manual vorgeschlagen, bei RT inkubiert.

Eine Elutionsverbesserung wurde bei einer Doppelelution vermutet. Bei drei Proben wurde das Eluat nach der letzten Zentrifugation ein weiteres Mal auf die Säule aufgetragen, 5 min inkubiert und danach zur Elution ein weiteres Mal zentrifugiert.

Test zur Verbesserung der Elution mittels verlängerter Inkubationszeit des Elutionspuffers Ein weiterer Parameter, der die Elution hätte verbessern können, war die Einwirkzeit des Elutionspuffers. Dabei wurde bei je drei Proben der Elutionspuffer 5 min, 10 min und 15 min lang bei Raumtemperatur inkubiert. Wegen sehr hoher Cp-Werte, die vermuten ließen, dass die verwendete CetuGEX-Probe keine DNA enthielt, wurde ein weiterer Versuch unter kontrollierter DNA-Zugabe durchgeführt. Dabei wurden als Proben jeweils 4 ml CetuGEX, versetzt mit 2 pg Standard -DNA, verwendet.

Mit dem QIAamp DNA Investigator Kit wurde im nächsten Schritt eine Versuchsreihe durchgeführt, welche zu einer besseren Elution der DNA der Silikamembran führen sollte. Es wurde damit versucht, die DNA-Ausbeuten zu maximieren. Zum einen wurde die Temperatur während der Inkubation des Elutionspuffers variiert. Die Inkubationen fanden entweder bei Raumtemperatur oder bei 45°C statt. Wie dazu in Tabelle 3 zu sehen ist, sind die Cp-Werte signifikant durch die erhöhte Temperatur gesunken.

**Tabelle 3:** Cp-Werte zur Verbesserung des Elutionsergebnisses mit dem QIAamp Investigator Kit mittels Temperatur. Die Elution wurde zweimal bei Raumtemperatur und zweimal bei 45°C durchgeführt. Als Probe wurde CetuGEX™ eingesetzt; Ø: Durchschnitt; σ Cp: Differenz Min gegen Max; rot: Ausreißer, der nicht berücksichtigt wurde.

|  | Raumtemperatur Probe 1 | Raumtemperatur Probe 2 | 45°C Probe 1 | 45°C Probe 2 |
|---|---|---|---|---|
| **Cp 1** | 33,75 | 32,70 | 31,03 | 31,51 |
| Cp 2 | 35,43 | 31,97 | 31,57 | 30,48 |
| **Cp 3** | 35,77 | 32,67 | 31,76 | 31,62 |
| Ø Cp | 34,98 | 32,45 | 31,45 | 31,57 |
| σ Cp | 1,08 | 0,41 | 0,38 | 0,08 |

Weiterhin vielversprechend zur Ausbeuteverbesserung erschien es, nach Eluieren der Probe das Eluat ein weiteres Mal auf die Säule zu geben (Doppelelution). Hierdurch sollte sich zusätzliche DNA von der Säule lösen. Doch die entsprechenden Cp-Werte bei Anwendung dieser Methode stiegen bzw. waren vergleichbar (siehe Tabelle 4), zeigten also keinen oder sogar einen nachteiligen Effekt an.

**Tabelle 4**: Cp-Werte der Elutionsverbesserung des QIAamp Investigator Kits mittels Doppelelution. Einfachelution: Elution einmal laut Manual. Bei der Doppelelution wurde das Eluat ein weiteres Mal auf die Säule aufgetragen sowie inkubiert und abzentrifugiert wie im Manual vorgegeben. Als Probe wurde CetuGEX™ eingesetzt; Ø: Durchschnitt; σ Cp: Standardabweichung.

| | Einfachelution Probe 1 | Einfachelution Probe 2 | Doppelelution Probe 1 | Doppelelution Probe 2 |
|---|---|---|---|---|
| Cp 1 | 34,56 | 33,55 | 34,49 | 40,00 |
| Cp 2 | 34,20 | 33,42 | 34,97 | 34,69 |
| Cp 3 | 34,83 | 33,61 | 34,76 | 33,82 |
| Ø Cp | 34,53 | 33,53 | 34,74 | 36,17 |
| σ Cp | 0,32 | 0,10 | 0,24 | 3,35 |

Als dritte Möglichkeit der Elutionsverbesserung kam in Frage, die Einwirkzeit des Elutionspuffers zu erhöhen. So wurden dazu Proben neben den im Protokoll vorgeschlagenen 5 min auch 10 und 15 Minuten lang inkubiert. In Tabelle 5 sind die Ergebnisse dieses Versuches dargestellt. Es lässt sich anhand der Ø Cp-Werte kein Trend erkennen.

**Tabelle 5**: Cp-Werte von den Versuchen zur Elutionsverbesserung des QIAamp Investigator Kits mittels längerer Einwirkzeit. Die Inkubation des Elutionspuffers auf der Säule wurde für 5 min, 10 min und 15 min bei Raumtemperatur durchgeführt. Als Probe wurde CetuGEX™ eingesetzt; Ø, σ Cp und rot: s. Legenden der vorangegangenen Tabellen.

| | 5 Minuten Probe 1 | 5 Minuten Probe 2 | 10 Minuten Probe 1 | 10 Minuten Probe 2 | 15 Minuten Probe 1 | 15 Minuten Probe 2 |
|---|---|---|---|---|---|---|
| Cp 1 | 37,01 | 36,72 | 37,25 | 37,41 | 35,86 | 37,93 |
| Cp 2 | 36,50 | 34,88 | 36,57 | 36,71 | 34,87 | 36,27 |
| Cp 3 | 35,80 | 34,97 | 36,40 | 36,81 | 34,05 | 36,12 |
| Ø Cp | 36,44 | 34,93 | 36,74 | 36,98 | 34,93 | 36,20 |
| σ Cp | 0,61 | 0,06 | 0,45 | 0,38 | 0,91 | 0,11 |

Da die Cp-Werte bei dem Versuch der Verbesserung der Elutionsbedingungen durch verlängerte Inkubationszeiten des Elutionspuffers mehrmals höher als 35,8 waren, war nicht auszuschließen, dass in dieser CetuGEX-Probe keine DNA vorhanden war. Um gesichert einen DNA-Nachweis durchzuführen, wurde aufgrund dessen der vorangegangene Versuch dahingehend modifiziert, dass das CetuGEX-Material mit 2 pg humaner DNA versetzt wurde (Konzentration des Spike also: 2 pg DNA pro 4 ml). Auch diese Ergebnisse (vgl. Tabelle 6), bezogen auf das Elutionsverhalten, waren vergleichbar mit denen ohne Zugabe von DNA (siehe Tabelle 6).

**Tabelle 6:** Cp-Werte von den Versuchen zur Elutionsverbesserung des QIAamp Investigator Kits mittels längerer Einwirkzeit. Die Inkubation des Elutionspuffers auf der Säule wurde jeweils für 5 min, 10 min und 15 min bei Raumtemperatur durchgeführt. Als Probe wurde CetuGEX™ + 2pg humaner DNA eingesetzt; ∅, σ Cp und rot: s. Legenden der vorangegangenen Tabellen.

| | 5 Minuten CetuGEX +2 pg humane DNA | 5 Minuten CetuGEX +2 pg humane DNA | 10 Minuten CetuGEX +2 pg humane DNA | 10 Minuten CetuGEX +2 pg humane DNA | 15 Minuten CetuGEX +2 pg humane DNA | 15 Minuten CetuGEX +2 pg humane DNA |
|---|---|---|---|---|---|---|
| Cp 1 | 33,49 | 35,54 | 34,86 | 35,97 | 33,16 | 36,14 |
| Cp 2 | 33,32 | 34,04 | 34,76 | 34,64 | 32,94 | 35,72 |
| Cp 3 | 33,07 | 34,64 | 34,47 | 34,20 | 32,84 | 35,05 |
| ∅ Cp | 33,29 | 34,74 | 34,70 | 34,94 | 32,98 | 35,64 |
| σ Cp | 0,21 | 0,75 | 0,20 | 0,92 | 0,16 | 0,55 |

Im Übrigen ist der Aufkonzentrierungsfaktor ca. 32-fach bei Verwendung dieses Kits.

**Beispiel 2**

1. Messen der Rest-DNA von FSH-Proben mit Hilfe von humaner qPCR

FSH-GEX™-Proben wurden unverdünnt, wie in Beispiel 1.1 beschrieben, vermessen. Um eventuelle Inhibitionen der PCR zu erkennen, wurde zusätzlich eine Inhibitionskontrolle durchgeführt. Dabei ist als Kontrollspike eine menschliche Standard-DNA-Konzentration von 90 fg/μL eingesetzt worden. Die Messungen wurden in Drei-fachbestimmungen mit der gleichen Probe an zwei verschiedenen Tagen durchgeführt, um dies Ergebnisse abzusichern.

Zum ersten Mal wurde mit Hilfe der humanen qPCR Rest-DNA von FSH-GEX™ quantifiziert. Wird eine Probenart zum ersten Mal vermessen, muss ausgetestet werden, ob und welche Probenvorbereitung für die Messung nötig ist. Die FSH-GEX™-Proben wurden zum Testen unverdünnt vermessen. Die Ergebnisse dazu sind in Tabelle 7 zu sehen.

**Tabelle 7**: Ergebnisse der Quantifizierung einer unverdünnten FSH-GEX™-Probe mit Hilfe von humaner qPCR; Die Messungen wurden an zwei unterschiedlichen Tagen durchgeführt; ∅ = Mittelwert; σ = Standardabweichung. Berechnung von ∅ Konz. siehe Text.

| | Cp | ∅ Cp | σ Cp | Gehalt DNA (fg) | ∅ Gehalt DNA (fg) | ∅ Konz. (fg/μL) |
|---|---|---|---|---|---|---|
| Messung 1 | 33,25 | 33,49 | 0,26 | 14,40 | 9,96 | 3,32 |
| | 33,77 | | | 5,17 | | |
| | 33,44 | | | 10,30 | | |
| Messung 2 | 36,19 | 36,87 | 0,61 | 2,88 | 1,49 | 0,50 |
| | 37,06 | | | 0,97 | | |
| | 37,37 | | | 0,63 | | |

Beispielrechnung des Mittelwertes der Konzentration bei Messung einer Probe:

Mittelwert Gehalt = 9,96 fg
Probenvolumen pro Vertiefung = 3 μl
Verdünnungsfaktor der Probe: 1

$$\varnothing \, \text{Konz.} = \frac{\varnothing \, \text{Gehalt}}{\text{Volumen}} \times \text{Verdünnungsfaktor} = \frac{9{,}96 \, \text{fg}}{3 \, \mu\text{l}} \times 1 = 3{,}32 \, \frac{\text{fg}}{\mu\text{l}}$$

Um mögliche Inhibitionen zu erkennen, wurde zusätzlich eine Inhibitionskontrolle durchgeführt (Ergebnisse siehe Tabelle 8).

**Tabelle 8**: Ergebnisse der Inhibititionskontrolle der Quantifizierung einer unverdünnten FSH-Probe mit Hilfe von humaner qPCR; die Messungen wurden an zwei unterschiedlichen Tagen durchgeführt; Ø = Mittelwert; σ = Standardabweichung; Berechnung von Ø Konz. Spike: siehe Text.

|  | Cp | Ø Cp | σ Cp | Gehalt DNA (fg) | Ø Gehalt Gesamt DNA (fg) | Ø Konz. Spike (fg/µL) |
|---|---|---|---|---|---|---|
| Messung 1 | 30,85 | 30,71 | 0,24 | 94,20 | 107,00 | 97,04 |
|  | 30,84 |  |  | 94,80 |  |  |
|  | 30,43 |  |  | 132,00 |  |  |
| Messung 2 | 31,99 | 31,95 | 0,08 | 78,90 | 81,30 | 79,81 |
|  | 32,00 |  |  | 78,60 |  |  |
|  | 31,85 |  |  | 86,40 |  |  |

Beispielrechnung des Mittelwertes der Konzentration Spike bei Inhibitionskontrolle:

Volumen des Spike = 1 µl
Gehalt an Gesamt-DNA = 107,00 fg
Gehalt, DNA der Probe (nicht der Inhibitionskontrolle) = 9,96 fg

$$\text{Ø Konz. Spike} = \frac{Gehalt\ Gesamt\ DNA - Gehalt\ DNA\ Probe}{Volumen\ Spike} = \frac{107{,}00\ fg - 9{,}96\ fg}{1\ \mu l} = 97{,}04\frac{fg}{\mu l}$$

Mit Hilfe der Inhibitionskontrolle wurde die Wiederfindung des Spikes errechnet. Es wurde festgelegt, dass eine PCR als erfolgreich angesehen wird, wenn die Wiederfindung 80 - 120% betrug. Tabelle 9 zeigt, dass die Wiederfindungsraten in diesem Rahmen lagen.

**Tabelle 9:** Wiederfindungsraten des Spikes der humanen qPCR von FSH-GEX™-Proben. Berechnungen siehe Text.

|  | Ø Konz. Spike (fg/µL) | Soll Spike (fg/µl) | Wiederfindung (%) | Soll Wiederfindung (%) |
|---|---|---|---|---|
| Probe 1 | 97,04 | 90,00 | 108, | 80 - 120 |
| Probe 2 | 79,81 | 90,00 | 89 | 80 - 120 |

Beispielrechnung der Wiederfindungsrate des Spike einer Inhibitionskontrolle:

Ø Konz. Spike = 97,04 fg/µl
Soll Spike = 90,00 fg/µl

$$Wiederfindung = \frac{\text{Ø Konz. Spike}}{Soll\ \text{Spike}} \times 100\% = \frac{97{,}04\frac{fg}{\mu l}}{90{,}00\frac{fg}{\mu l}} \times 100\% = 108\%$$

**Beispiel 3**

Aufnahme der Standardkurve und Reproduzierbarkeit

Tabelle 10 zeigt die bei der Aufnahme der Standardkurve erhaltenen Cp-Werte (Dreifachbestimmung) für die jeweiligen DNA-Konzentrationen der E1-DNA der 5 unterschiedlichen Messungen.

**Tabelle 10: Reproduzierbarkeit der E1-DNA als Standard**Dargestellt sind die Cp-Werte der 5 Messungen (jeweils Dreifachbestimmung) der E1-DNA der Konzentrationen 27,5 ng/µL bis 2,75 fg/µL und der NTC unter optimierten qPCR Bedingungen; rot markiert sind Ausreißer, die zur Berechnung des Mittelwertes ausgeschlossen wurden; Ausreißer wurden als solche definiert, da sie stark vom zu erwartenden als auch von den anderen Messungen erhaltenen Cp-Werten abwichen; der vorletzten Spalte ist der Mittelwert (∅) aller Cp-Werte, bei der in Spalte 1 aufgeführten E1-DNA Konzentration, zu entnehmen; in der letzten Spalte ist die aus den erhaltenen Cp-Werten berechnete Standardabweichung (STABW) aufgeführt

| Konzentration E1-DNA | Cp-Werte Messung 1 | Cp-Werte Messung 2 | Cp-Werte Messung 3 | Cp-Werte Messung 4 | Cp-Werte Messung 5 | ∅ Cp-Werte | STABW |
|---|---|---|---|---|---|---|---|
| 27,5 ng/µL | 10,60 | 10,50 | 10,52 | 10,58 | 10,51 | 10,58 | 0,05 |
| | 10,61 | 10,63 | 10,59 | 10,59 | 10,55 | | |
| | 10,62 | 10,56 | 10,52 | 10,71 | 10,57 | | |
| 2,75 ng/µL | 13,93 | 13,91 | 13,96 | 13,92 | 13,93 | 13,92 | 0,04 |
| | 13,95 | 13,91 | 13,95 | 13,89 | 13,86 | | |
| | 13,91 | 13,93 | 14,02 | 13,92 | 13,88 | | |
| 275 pg/µL | 17,55 | 17,51 | 17,49 | 17,52 | 17,48 | 17,50 | 0,03 |
| | 17,50 | 17,54 | 17,52 | 17,49 | 17,49 | | |
| | 17,54 | 17,48 | 17,49 | 17,50 | 17,46 | | |
| 27,5 pg/µL | 21,03 | 21,00 | 21,04 | 20,97 | 21,02 | 21,02 | 0,04 |
| | 21,09 | 21,08 | 21,04 | 20,96 | 21,02 | | |
| | 21,07 | 21,00 | 21,03 | 21,01 | 20,99 | | |
| 2,75 pg/µL | 24,67 | 24,56 | 24,63 | 24,54 | 24,58 | 24,59 | 0,04 |
| | 24,64 | 24,62 | 24,55 | 24,53 | 24,58 | | |
| | 24,54 | 24,54 | 24,64 | 24,58 | 24,61 | | |
| 275 fg/µL | 28,05 | 28,06 | 28,12 | 28,13 | 28,51 | 28,27 | 0,28 |
| | 28,23 | 28,12 | 28,06 | 28,47 | 28,52 | | |
| | 27,95 | 28,13 | 28,18 | 28,43 | 29,02 | | |
| 27,5 fg/µL | 31,65 | 31,86 | 31,64 | 31,50 | 31,64 | 31,76 | 0,31 |
| | 31,99 | 31,64 | 31,81 | 31,79 | 31,82 | | |
| | 31,55 | 32,42 | 32,22 | 31,11 | 31,81 | | |
| 2,75 fg/µL | 35,00 | 35,05 | 35,8 | 33,82 | 34,84 | 35,27 | 0,69 |
| | 35,00 | 36,15 | 40 | 35,03 | 35,90 | | |
| | 35,00 | 35,41 | 30,45 | 34,68 | 35,55 | | |
| NTC | / | / | 40 | 36,06 | / | 38,04 | 2,16 |
| | 35 | 38,1 | 40 | 36,04 | / | | |
| | / | / | / | / | / | | |

Im Bereich von 27,5 ng/µL bis zu 2,75 pg/µL sind die Cp-Werte sowohl innerhalb der Dreifachbestimmung als auch zwischen den einzelnen Messungen gut reproduzierbar. Tabelle 11 zeigt, dass auch die Cp-Werte der Konzentrationen 275 fg/µL bis 2,75 fg/µL noch mit akzeptablen Schwankungen reproduzierbar sind.

**Tabelle 11: Zusammenfassung der Reproduzierbarkeit der E1-DNA inklusive der Standardabweichung**Dargestellt sind die Cp-Mittelwerte inklusive der aus den Messwerten resultierende Standardabweichung

| Konzentration E1-DNA | log DNA-Konzentration | Ø Cp-Werte | Standardabweichung |
|---|---|---|---|
| 27500000 fg/µL | 7,44 | 10,58 | 0,05 |
| 2750000 fg/µL | 6,44 | 13,92 | 0,04 |
| 275000 fg/µL | 5,44 | 17,50 | 0,03 |
| 27500 fg/µL | 4,44 | 21,02 | 0,04 |
| 2750 fg/µL | 3,44 | 24,59 | 0,04 |
| 275 fg/µL | 2,44 | 28,27 | 0,28 |
| 27,5 fg/µL | 1,44 | 31,76 | 0,31 |
| 2,75fg/µL | 0,44 | 35,27 | 0,69 |
| NTC | | 38,04 | 2,16 |

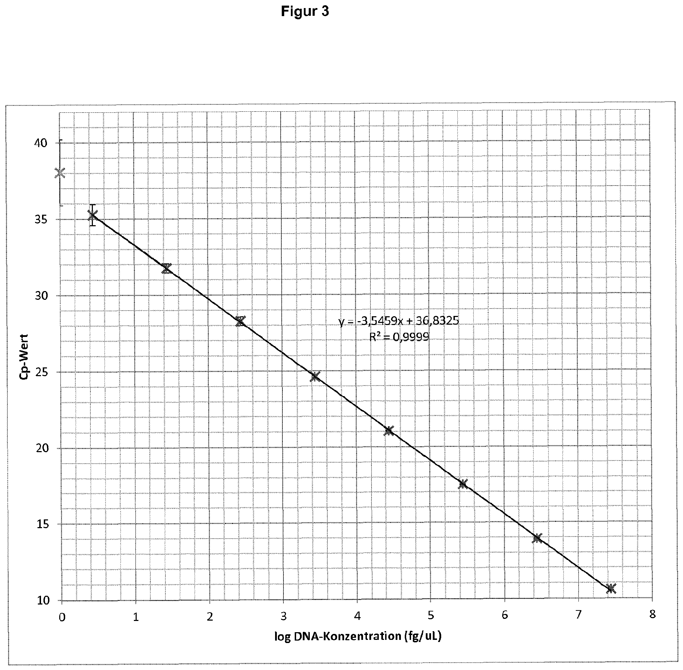

Figur 3 zeigt die über Excel erstellte Standardgerade zu Tabelle 11. Hierbei ist der Logarithmus der jeweiligen E1-DNA-Konzentration gegen den jeweiligen Mittelwert, inklusive Standardabweichung, aufgetragen.

Das Bestimmtheitsmaß von $R^2 \geq 0,99$ verdeutlicht den linearen Zusammenhang von der E1-DNA zu den Cp-Werten. Die Effizienz der Standardkurve lässt sich anhand folgender Formel berechnen:

$$E = 10^{\frac{-1}{\text{Steigung}}}$$

Die Steigung der von Excel berechneten Standardgeraden beträgt -3,5459. Somit ergibt sich für die Effizienz:

$$E = 10^{\frac{-1}{-3,5459}}$$

$$E = 1,914$$

Die Standardgerade liegt mit einer Effizienz von 1,914 in dem von Roche als akzeptabel eingestuften Bereich ($\geq 1,8$). Somit ist die E1-DNA als Standard und zur Aufnahme einer Standardgeraden geeignet. Es ist jedoch wichtig zu definieren, in welchem Bereich die Standardabweichung der jeweiligen Konzentration liegen darf, um noch von einer eindeutigen Quantifizierbarkeit auszugehen. Am LightCycler480 wurde schließlich eine Standardgerade durch die Messung von 6 Replikaten pro Konzentration (27,5 ng/µL bis 2,75fg/µL) aufgenommen. Die erhaltenen Cp-Werte sind inklusive Standardabweichung in Tabelle 12 dargestellt.

# EP 3 198 011 B1

**Tabelle 12: Werte zur Aufnahme der Standardkurve mit E1-DNA am LightCycler480** Dargestellt sind die gemessenen Cp-Werte bei der jeweiligen Konzentration, der daraus resultierende Mittelwert und die Standardabweichung; bei rot markierten Werten handelt es sich um Ausreißer (Ausreißertest nach Grubbs), die zur Berechnung des Mittelwerts, der Standardabweichung und der Erstellung der Standardgeraden herausgelassen wurden

| Konzentration E1-DNA | Cp-Werte | | Ø Cp-Werte | Standardabweichung |
|---|---|---|---|---|
| 27,5 ng/µL | 10,30 | 10,30 | 10,30 | 0,02 |
| | 10,33 | 10,25 | | |
| | 10,31 | 10,29 | | |
| 2,75 ng/µL | 13,70 | 13,72 | 13,75 | 0,03 |
| | 13,76 | 13,80 | | |
| | 13,74 | 13,75 | | |
| 275 pg/µL | 17,23 | 17,22 | 17,24 | 0,03 |
| | 17,20 | 17,22 | | |
| | 17,28 | 17,26 | | |
| 27,5 pg/µL | 20,80 | 20,82 | 20,82 | 0,03 |
| | 20,77 | 20,88 | | |
| | 20,80 | 20,83 | | |
| 2,75 pg/µL | 24,34 | 24,43 | 24,39 | 0,06 |
| | 24,49 | 24,41 | | |
| | 24,32 | 24,33 | | |
| 275 fg/µL | 27,74 | 28,07 | 28,03 | 0,02 |
| | 28,02 | 28,04 | | |
| | 28,03 | 28,00 | | |
| 27,5 fg/µL | 31,85 | 32,03 | 31,73 | 0,30 |
| | 31,95 | 31,80 | | |
| | 31,61 | 31,12 | | |
| 2,75 fg/µL | 36,17 | 33,18 | 34,75 | 0,89 |
| | 35,00 | 34,56 | | |
| | 34,50 | 35,10 | | |
| NTC | 35,54 | Unknown | | |
| | Unknown | 37,19 | | |
| | 40 | 36,96 | | |

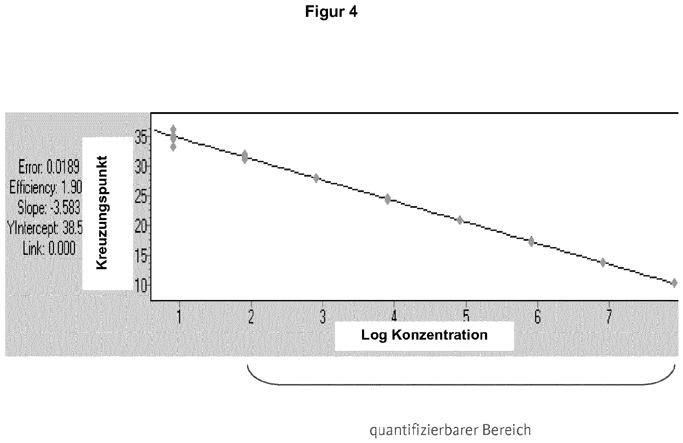

Figur 4 zeigt die von der LigthCycler480 Software berechnete und abgespeicherte Standardkurve. Bei einer akzeptablen Standardabweichung von 0,45 zur Quantifizierung ergibt sich eine Quantifizierbarkeit im Bereich von 27,5 ng/µL bis 27,5 fg/µL und eine Nachweisbarkeit bis 2,75 fg/µL.

Als kommerziell erhältliches Kit zum Nachweis und zur Quantifizierung humaner DNA existiert beispielsweise das "Investigator Quantiplex Kit" von Qiagen (Nachweisgrenze ∼ 1 pg/µL, Quantifizierungsgrenze 4,9 pg/µL), wobei ein 146 bp Fragment einer autosomalen multi-copy Region des humanen Genoms amplifiziert wird. Weiter wird von Promega das "Plexor HY System" angeboten, wobei eine Quantifizierungsgrenze von 3,2 pg/µL angegeben ist.

Weitere bekannte Assays zum Nachweis und der Quantifizierung von Wirts-Zell DNA ist z.B. der Picogreen Assay, bei welchem nicht sequenzspezifisch über Fluoreszenz doppelsträngige DNA nachgewiesen wird. Die Nachweisgrenze liegt hier bei ∼ 1 pg/µL.

Es existieren keine Veröffentlichungen oder kommerzielle Kits, in denen eine so geringere Nachweisgrenze, wie der hier etablierten, vorgestellt wird. Selbst die Quantifizierungsgrenze der hierin etablierten qPCR liegt mit 27,5 fg/μL noch um > 33-fach unterhalb der, in kommerziell erhältlichen Kits oder anderen bekannten Methoden. Diese Nachweisgrenzen konnten zudem bereits ohne den Aufkonzentrierungseffekt der DNA-Präparation unterschritten werden. Dieser hierin etablierte Schritt senkt die Nachweisgrenze der in den Ausgangsproben enthaltenen DNA weiter um ein Vielfaches.

Eine weitere Erhöhung der Sensitivität ergibt sich durch den Aufkonzentrierungsschritt - ca. 32-fach. Somit würde im kombinierten Assay (Protease Spaltung, Aufkonzentrierung und Anreichern der Nukleinsäure) eine Nachweisgrenze für die Ausgangskonzentration an Nukleinsäure, insbesondere DNA, in einer Probe von mindestens 0,086 fg/μL und eine Quantifizierungsgrenze von 0,86 fg/μL erreicht werden.

## Beispiel 4

In diesem Experiment handelt es sich um eine Bestätigung, dass das erfindungsgemäße Verfahren die gewünschten Resultate erbringt. Es werden folgende Versuchsansätze unternommen:

- Komplette Prozedur (KP)
- Prozedur ohne Protease-Schritt (w/o PK)
- Prozedur ohne Konzentrierungs-Schritt (w/o UF)
- Prozedur ohne Anreichern der Nukleinsäure (w/o SM)

Für jeden Versuchsansatz werden zwei unterschiedliche Mengen genomischer K562-DNA dem Produkt zugespiked (finale Spike-Konzentrationen: 0.4 pg/mL und 40 pg/mL). Es soll gezeigt werden, dass nur die komplette Prozedur ein zuverlässiges Signal für 0.4 pg/mL erzielen kann.

## Material

15 mL Falcon-Tubes
Parafilm
Amicon Ultrafiltrationseinheiten
Mikrotiterplatten (96-Well)
Abdeckfolie für 96-Well Platte

## Geräte

Wasserbad bei 56°C
Zentrifuge Rotina von Hettich
LightCycler 480 von Roche

## Reagenzien

CHO K1 V2-Standard DNA (2 pg/uL)
K-652 DNA V2-Standard (900 pg/uL)
Proteinase K mit 923 U / mL
10%iges SDS
PCR-Materialien
SYBR-Mix
Primer Yb8F (10uM), Yb8R (10uM)
Primer CHO1F (100uM); CHO1R (100uM)
PCR-H$_2$0
QIAgen DNS Investigator kit (Tag2)
Puffer AW1 (in aliquots)
Puffer AW2 (in aliquots)
Elutionspuffer

## Erstellung einer K-562-DNA Verdünnungreihe

Es werden K-562 DNA-Verdünnungsreihen hergestellt: 100 pg/uL, 10 pg/uL, 1 pg/uL, 250 fg/uL, 100 fg/uL, 50 fg/uL, 25 fg/uL und 10 fg/uL

**Spiking**

- Spiking wird gemäß Tabelle 13 durchgeführt

- 3,2 uL der genomischen CHO DNA (V5-Verdünnung) werden hinzugegeben

- Unterschiedliche Mengen des K562-Standards werden hinzugegeben

- Es wird eine Kontrolle mit CHO-DNA Spike durchgeführt.

### Tabelle 13 – DNA Spiking Übersicht

| | Komplette Prozedur (KP) – 0.4 pg/mL | Komplette Prozedur (KP) – 40 pg/mL | W/O PK – 0.4 pg/mL | W/O PK – 40 pg/mL |
|---|---|---|---|---|
| Probenvolumen | 4 mL | 4 mL | 4 mL | 4 mL |
| DNA Standard Konz. | 250 fg/uL | 10 pg/uL | 250 fg/uL | 10 pg/uL |
| Zugabe DNA-Standard | 6,4 uL | 16 uL | 6,4 uL | 16 uL |
| Zugabe CHO-Spike DNA V5 (1 pg/uL) | 3,2 uL (-> 0,8 pg/mL) | 3,2 uL (-> 0,8 pg/mL) | 3,2 uL (-> 0,8 pg/mL) | 3,2 uL (-> 0,8 pg/mL) |
| Finale DNA Konz. | 0,4 pg/mL (K562) + 0,8 pg/mL (Spike) | 40 pg/mL (K562) + 0,8 pg/mL (Spike) | 0,4 pg/mL (K562) + 0,8 pg/mL (Spike) | 40 pg/mL (K562) + 0,8 pg/mL (Spike) |

### Tabelle 13 – DNA Spiking Übersicht

| | W/O UF – 0.4 pg/mL | W/O UF – 40 pg/mL | W/O SM – 0.4 pg/mL | W/O SM – 40 pg/mL |
|---|---|---|---|---|
| Probenvolumen | 4 mL | 4 mL | 4 mL | 4 mL |
| DNA Standard Konz. | 250 fg/uL | 10 pg/uL | 250 fg/uL | 10 pg/uL |
| Zugabe DNA-Standard | 6,4 uL | 16 uL | 6,4 uL | 16 uL |
| Zugabe CHO-Spike DNA V5 (1 pg/uL) | 3,2 uL (-> 0,8 pg/mL) | 3,2 uL (-> 0,8 pg/mL) | 3,2 uL (-> 0,8 pg/mL) | 3,2 uL (-> 0,8 pg/mL) |
| Finale DNA Konz. | 0,4 pg/mL (K562) + 0,8 pg/mL (Spike) | 40 pg/mL (K562) + 0,8 pg/mL (Spike) | 0,4 pg/mL (K562) + 0,8 pg/mL (Spike) | 40 pg/mL (K562) + 0,8 pg/mL (Spike) |

**Proteinase K Verdau**

Proteinase K-Verdau erfolgt gemäß dem Fachmann bekannten Protokollen.

**Ultrafiltration**

Ultrafiltration erfolgt gemäß dem Fachmann bekannten Protokollen.

**Nukleinsäure-Aufreinigung**

DNA-Aufreinigung erfolgt mittels Silika-Adsorption, z.B. mit QIAamp DNA Invest. Kit nach Clean Up Protokoll

**Nukleinsäure-Nachweis**

| | | | |
|---|---|---|---|
| Pre-Inc.: | 1 Zyklus | Analysis Mode: none | |
| Amplification: | 45 Zyklen | Analysis Mode: Quantification | |
| Melting Curve: | 1 Zyklus | Analysis Mode: Melting Curves | |
| Cooling: | 1 Zyklus | Analysis Mode: None | |

| | Target °C | Acqu. Mode | Hold | Ramp R. | Acqu.per°C |
|---|---|---|---|---|---|
| Pre-Inc.: | 95°C | none | 10 min. | 4,4°C/s | - |
| Amplification: | 95°C | none | 5 s | 4,4°C/s | - |
| | 71°C | single | 20 s | 2,2°C/s | - |
| Melting Curve: | 95°C | none | 5 s | 4,4°C/s | - |
| | 65°C | none | 1 min. | 2,2°C/s | - |
| | 97°C | continuous | - | | 7 acqu./°C |
| Cooling: | 40°C | none | 10 s | 1,5°C/s | - |

**Ergebnisse und Auswertung**

**Tabelle 14: qPCR Messung und DNA-Bestimmung**

| NV - nicht verfügbar; da keine Daten vorhanden (kein Signal) | | | | | | | |
|---|---|---|---|---|---|---|---|
| Proben-Nr. | Bezeichnung | qPCR Messung [pg/mL] | Konz.-Faktor [-] | **Bestimmte DNA Konzentration [pg/mL]** | Soll | Wiederfindung [%] |
| 001 | CM + 40 pg/mL KP | 1720 | 62,5 | **27,5** | 40,0 | 68,8 |
| 002 | CM + 40 pg/mL W/O PK | NV | 25 | **NV** | 40,0 | 0,0 |
| 003 | CM + 40 pg/mL W/O UF | 222 | 6,25 | **35,5** | 40,0 | 88,8 |
| 004 | CM + 40 pg/mL W/O SM | NV | 10 | **NV** | 40,0 | 0,0 |
| 005 | CM + 0,4 pg/mL KP | 25,4 | 62,5 | **~0,4** | 0,4 | ~100% |
| 006 | CM + 0,4 pg/mL w/o PK | NV | 25 | **NV** | 0,4 | 0,0 |
| 007 | CM + 0,4 pg/mL w/o UF | NV | 6,25 | **NV** | 0,4 | 0,0 |
| 008 | CM + 0,4 pg/mL w/o SM | NV | 10 | **NV** | 0,4 | 0,0 |

Zwei der vier getesteten Prozeduren ermöglichen eine DNA Messung mittels qPCR, nämlich die komplette Prozedur sowie die Prozedur ohne Anwendung der Ultrafiltrations-Einheiten (Amicon Ultra 4 mL, Merck Millipore). Bei einer K562-DNA Spike-Konzentration von 40 pg/mL erreicht man 69% Wiederfindung, bei einer Konzentrierung von 62,5-fach unter Anwendung der kompletten Prozedur. Bei ausbleibender Ultrafiltration liegt die Wiederfindung bei etwa 89%, wobei der Konzentrationsfaktor aber nur bei 6,25-fach liegt und somit 10-fach geringer ist. Das Auslassen des Proteinase K-Schritts oder der Aufreinigung mittels Silikamembran (z.B. QIAamp DNA Investigator Kit) hat zur Folge, dass keine Messung zustande kommt. Bei einer K562-DNA Konzentration von 0,4 pg/mL in einer CetuGEX-Antikörperpräparation ist eine Messung nur mit der kompletten Prozedur möglich. Durch die Prozedur und eine 62,5-fache Konzentrierung wird eine qPCR Messung bei 25,4 pg/mL (Gemittelter Cp-Wert: 31,84) ermöglicht, was etwa einer 100%igen Wiederfindung entspricht.

**Tabelle 15: Inhibitionslevel der Probenmatrizes nach der Aufreinigung.**

| Zero Inhibition Control (ZIC): 291 pg/mL; Sollwert entspricht der Summe aus ZIC und der jeweiligen Probenmessung; IK - Inhibitionskontrolle; NV - nicht verfügbar; keine Daten verfügbar; j -ja; n - nein | | | | | |
|---|---|---|---|---|---|
| IK zu Proben-Nr. | Bezeichnung der IK | qPCR Messung der IK [pg/mL] | Soll [pg/mL] | Wiederfindung [%] | Inhibition (j/n) über 30% |
| 001 | IK CM+ 40 pg/mL KP | 2080 | 2011 | 103 | n |
| 002 | IK CM + 40 pg/mL W/O PK | NV | 291 | NV | j |
| 003 | IK CM + 40 pg/mL W/O UF | 613 | 513 | 119 | n |
| 004 | IK CM + 40 pg/mL W/O SM | NV | 291 | NV | j |
| **005** | **IK CM + 0,4 pg/mL KP** | **408** | **316** | **129** | **n** |
| 006 | IK CM + 0,4 pg/mL w/o PK | NV | 291 | NV | j |
| 007 | IK CM + 0,4 pg/mL w/o UF | 354 | 291 | 121 | n |
| 008 | IK CM + 0,4 pg/mL w/o SM | NV | 291 | NV | j |

Die Inhibitionslevels der Proben wurden anhand eines PCR-basierten K562 DNA-Spikes (Inhibitionskontrolle; ~333 pg/mL) geprüft. Bei der kompletten Prozedur sowie der Prozedur ohne Ultrafiltration ist die Wiederfindung der Inhibitionskontrolle zwischen 70 und 130%, womit deutlich keine Inhibition vorliegt. Das Auslassen des Proteinase K-Schritts oder der Aufreinigung mittels Silikamembran hinterlässt inhibitorische Substanzen in der aufgereinigten Probe.

**Patentansprüche**

1. Verfahren zum Nachweis einer Nukleinsäure in einer Probe, die biologisches Material enthält, umfassend

   (a) Behandeln der Probe mit Protease;
   (b) Konzentrieren der Probe;
   (c) Anreichern der Nukleinsäure aus der Probe durch Affinitätschromatographie durch Silika-Adsorption; und
   (d) Nachweisen der Nukleinsäure mittels quantitativer PCR,

   wobei das biologische Material Antikörper oder ein therapeutisches Protein enthält.

2. Verfahren nach Anspruch 1, wobei das biologische Material aus Säugerzellen, Bakterienzellen oder Pilzzellen stammt.

3. Verfahren nach einem der vorhergehenden Ansprüche, wobei das biologische Material Nukleinsäuren, Proteine, Kohlenhydrate und/oder Fette enthalten kann, wobei die Nukleinsäuren vorzugsweise DNA oder RNA sein können, und/oder wobei die Nukleinsäure von einer Wirtszelle stammen kann, die das biologische Material herstellt.

4. Verfahren nach einem der vorhergehenden Ansprüche, wobei der Antikörper ein Antikörper gegen EGFR, Her2, TA-MUC1, TF oder LeY ist, und/oder wobei das Protein FSH, hCG, hLH, hGH, Factor VII, Factor FVIIa, Factor FVIII, Factor VIIIa, Factor IX, Factor IXa, Factor X, oder Factor Xa ist.

5. Verfahren nach einem der vorhergehenden Ansprüche, wobei die Säugerzellen menschliche Zellen, Primatenzellen, Mauszellen, Rattenzellen, Hamsterzellen, oder Hasenzellen sind, wobei die Säugerzellen vorzugsweise PER.C6, HEK-Zellen, NS0-Zellen, Vero-Zellen, CHO-Zellen, beispielsweise DUXB11, DG44, oder CHOK1, Maus-Hybridomzellen, Ratten-Hybridomzellen, oder Hasen-Hybridomzellen sind.

6. Verfahren nach einem der vorhergehenden Ansprüche, wobei die Probe flüssiges biologisches Material oder in Flüssigkeit gelöstes oder suspendiertes biologisches Material ist.

7. Verfahren nach einem der vorhergehenden Ansprüche, wobei die Protease in Schritt (a) Proteinase K ist, und/oder wobei das Konzentrieren der Probe in Schritt (b) mittels Filtration unter Volumenreduktion erfolgt, wobei die Filtration vorzugsweise eine Ultrafiltration ist.

8. Verfahren nach Anspruch 1, wobei die Silika-Adsorption mittels einer Silika-basierten Membran erfolgt.

9. Verfahren nach einem der vorhergehenden Ansprüche, wobei Nachweisen der Nukleinsäure mittels quantitativer PCR in Schritt (d) auf repetitive Elemente abstellt.

10. Verfahren nach Anspruch 9, wobei die repetitiven Elemente vorzugsweise Alu-Sequenzen oder Alu-äquivalente Sequenzen sind.

11. Verfahren nach einem der vorhergehenden Ansprüche, wobei im Schritt (d) die Primerpaare mit der SEQ ID Nr. 1 und 2, SEQ ID Nr. 3 und SEQ ID Nr. 4, SEQ ID Nr. 5 und 6, SEQ ID Nr. 1 und 7, SEQ ID Nr.1 und 8, SEQ ID Nr.1 und 9, SEQ ID Nr.1 und 10, SEQ ID Nr. 11 und 12 oder ein Gemisch aus den Primerpaaren mit den SEQ ID Nr. 1 und 7-10 verwendet werden.

12. Verfahren nach einem der vorhergehenden Ansprüche, wobei die Probe ein biopharmazeutisches oder biotechnologisches Produkt ist.

13. Verfahren nach einem der vorhergehenden Ansprüche, wobei der Nachweis einer Nukleinsäure in der Probe quantitativ ist.

14. Verfahren nach einem der vorhergehenden Ansprüche, wobei das Verfahren zum Nachweis einer Nukleinsäure einer Wirtszelle in biologischem Material ist, das an Menschen verabreicht wird, und/oder

wobei das Verfahren zur Bestimmung ist, ob biologisches Material zur Verabreichung an Menschen im Wesentlichen frei von Wirtszell-Nukleinsäuren, insbesondere DNA ist, und/oder

wobei das Verfahren zum Quantifizieren von Wirtszell-Nukleinsäuren in einer Probe ist.

15. Kit zum Durchführen eines Verfahrens nach einem der Ansprüche 1 bis 14, umfassend

(a) Protease;
(b) Mittel zum Konzentrieren von flüssigen Proben;
(c) Mittel für die Affinitätschromatographie basierend auf Silika-Adsorption und
(d) DNA-Polymerase und ein Primerpaar mit den SEQ ID Nr. 1 und 2, SEQ ID Nr. 3 und SEQ ID Nr. 4, SEQ ID Nr. 5 und 6, SEQ ID Nr. 1 und 7, SEQ ID Nr.1 und 8, SEQ ID Nr.1 und 9, SEQ ID Nr.1 und 10, SEQ ID Nr. 11 und 12 oder ein Gemisch aus den Primerpaaren mit den SEQ ID Nr. 1 und 7-10 zum Amplifizieren von repetitiven Elementen in DNA.

## Claims

1. A method for detecting a nucleic acid in a sample containing biological material, comprising

(a) treating the sample with protease;
(b) concentrating the sample;
(c) enriching the nucleic acid from the sample by affinity chromatography by silica adsorption; and
(d) detecting the nucleic acid by means of quantitative PCR,

wherein the biological material contains antibodies or a therapeutic protein.

2. The method according to claim 1, wherein the biological material originates from mammalian cells, bacterial cells or fungal cells.

3. The method according to any one of the preceding claims, wherein the biological material can contain nucleic acids, proteins, carbohydrates and/or fats, wherein the nucleic acids can preferably be DNA or RNA, and/or wherein the nucleic acid can originate from a host cell which produces the biological material.

4. The method according to any one of the preceding claims, wherein the antibody is an antibody against EGFR, Her2, TA-MUC1, TF or LeY, and/or wherein the protein is FSH, hCG, hLH, hGH, Factor VII, Factor FVIIa, Factor FVIII, Factor VIIIa, Factor IX, Factor IXa, Factor X, or Factor Xa.

5. The method according to any one of the preceding claims, wherein the mammalian cells are human cells, primate cells, mouse cells, rat cells, hamster cells, or rabbit cells,

wherein the mammalian cells are preferably PER.C6, HEK cells, NS0 cells, Vero cells, CHO cells, for example, DUXB11, DG44, or CHOK1, mouse hybridoma cells, rat hybridoma cells, or rabbit hybridoma cells.

6. The method according to any one of the preceding claims, wherein the sample is liquid biological material or biological material dissolved or suspended in liquid.

7. The method according to any one of the preceding claims, wherein the protease in step (a) is Proteinase K, and/or wherein concentrating the sample in step (b) occurs by means of filtration under volume reduction, wherein the filtration is preferably an ultrafiltration.

8. The method according to claim 1, wherein the silica adsorption occurs by means of a silica-based membrane.

9. The method according to any one of the preceding claims, wherein detecting the nucleic acid by means of quantitative PCR in step (d) focuses on repetitive elements.

10. The method according to claim 9, wherein the repetitive elements are preferably Alu sequences or Alu-equivalent sequences.

11. The method according to any one of the preceding claims, wherein in step (d) the primer pairs with SEQ ID NOs. 1 and 2, SEQ ID NO. 3 and SEQ ID NO. 4, SEQ ID NOs. 5 and 6, SEQ ID NOs. 1 and 7, SEQ ID NOs. 1 and 8, SEQ ID NOs. 1 and 9, SEQ ID NOs. 1 and 10, SEQ ID NOs. 11 and 12 or a mixture of the primer pairs with SEQ ID NOs. 1 and 7-10 are used.

12. The method according to any one of the preceding claims, wherein the sample is a biopharmaceutical or biotechnological product.

13. The method according to any one of the preceding claims, wherein the detection of a nucleic acid in the sample is quantitative.

14. The method according to any one of the preceding claims, wherein the method is for detecting a nucleic acid of a host cell in biological material that is administered to humans, and/or
wherein the method is for determining whether biological material for administration to humans is essentially free of host cell nucleic acids, in particular DNA, and/or
wherein the method is for quantifying host cell nucleic acids in a sample.

15. A kit for carrying out a method according to any one of claims 1 to 14, comprising

   (a) protease;
   (b) means for concentrating liquid samples;
   (c) means for the affinity chromatography based on silica adsorption and
   (d) DNA polymerase and a primer pair with SEQ ID NOs. 1 and 2, SEQ ID NO. 3 and SEQ ID NO. 4, SEQ ID NOs. 5 and 6, SEQ ID NOs. 1 and 7, SEQ ID NOs. 1 and 8, SEQ ID NOs. 1 and 9, SEQ ID NOs. 1 and 10, SEQ ID NOs. 11 and 12, or a mixture of the primer pairs with SEQ ID NOs. 1 and 7-10 for amplifying repetitive elements in DNA.

**Revendications**

1. Procédé de détection d'un acide nucléique dans un échantillon contenant du matériel biologique comportant

   (a) traiter l'échantillon avec de la protéase;
   (b) concentrer l'échantillon;
   (c) enrichir l'acide nucléique de l'échantillon par la chromatographie d'affinité par adsorption de la silice; et
   (d) détecter l'acide nucléique par la PCR quantitative,

   le matériel biologique contenant des anticorps ou une protéine thérapeutique.

2. Procédé selon la revendication 1, le matériel biologique étant dérivé des cellules mammalienne, des cellules bactériennes ou des cellules fongiques.

3.  Procédé selon l'une quelconque des revendications précédentes, le matériel biologique pouvant contenir des acides nucléiques, des protéines, des glucides et/ou des matières grasses, les acides nucléiques pouvant être de préférence l'ADN ou l'ARN, et/ou l'acide nucléique pouvant être dérivé d'une cellule hôte qui produit le matériel biologique.

4.  Procédé selon l'une quelconque des revendications précédentes, l'anticorps étant un anticorps contre EGFR, Her2, TA-MUC1, TF ou LeY, et/ou la protéine étant FSH, hCG, hLH, hGH, le facteur VII, le facteur FVIIa, le facteur FVIII, le facteur VIIIa, le facteur IX, le facteur IXa, le facteur X ou le facteur Xa.

5.  Procédé selon l'une quelconque des revendications précédentes, les cellules mammaliennes étant des cellules humaines, des cellules de primate, des cellules murines, des cellules de rat, des cellules de hamster ou des cellules de lièvre,
    les cellules mammaliennes étant de préférence PER.C6, des cellules HEK, des cellules NS0, des cellules Vero, des cellules CHO, par exemple, DUXB11, DG44 ou CHOK1, des cellules d'hybridomes murins, des cellules d'hybridomes de rat ou des cellules d'hybridomes de lièvre.

6.  Procédé selon l'une quelconque des revendications précédentes, l'échantillon étant du matériel biologique liquide ou du matériel biologique dissous ou en suspension dans un liquide.

7.  Procédé selon l'une quelconque des revendications précédentes, la protéase dans l'étape (a) étant la protéinase K, et/ou la concentration de l'échantillon dans l'étape (b) ayant lieu par filtration sous réduction du volume, la filtration étant de préférence une ultrafiltration.

8.  Procédé selon la revendication 1, l'adsorption de la silice ayant lieu par une membrane sur base de silice.

9.  Procédé selon l'une quelconque des revendications précédentes, la détection de l'acide nucléique par la PCR quantitative dans l'étape (d) reposant sur des éléments répétitifs.

10. Procédé selon la revendication 9, les éléments répétitifs étant de préférence des séquences alu ou des séquences équivalents à Alu.

11. Procédé selon l'une quelconque des revendications précédentes, dans l'étape (d) les paires d'amorces avec SEQ ID NO. 1 et 2, SEQ ID NO. 3 et SEQ ID NO. 4, SEQ ID NO. 5 et 6, SEQ ID NO. 1 et 7, SEQ ID NO. 1 et 8, SEQ ID NO. 1 et 9, SEQ ID NO. 1 et 10, SEQ ID NO. 11 et 12 ou un mélange des paires d'amorces avec SEQ ID NO. 1 et 7-10 étant utilisées.

12. Procédé selon l'une quelconque des revendications précédentes, l'échantillon étant un produit biopharmaceutique ou biotechnologique.

13. Procédé selon l'une quelconque des revendications précédentes, la détection d'un acide nucléique dans l'échantillon étant quantitative.

14. Procédé selon l'une quelconque des revendications précédentes, le procédé étant pour détecter un acide nucléique d'une cellule hôte dans du matériel biologique qui est administré à l'homme, et/ou le procédé étant pour déterminer si du matériel biologique pour l'administration à l'homme est sensiblement exempt des acides nucléiques de cellule hôte, surtout de l'ADN, et/ou
    le procédé étant pour quantifier des acides nucléiques de cellule hôte dans un échantillon.

15. Kit pour effectuer un procédé selon l'une quelconque des revendications 1 à 14, comportant

    (a) la protéase;
    (b) des moyens pour concentrer des échantillons liquides;
    (c) des moyens pour la chromatographie d'affinité basée sur l'adsorption de la silice et
    (d) l'ADN-polymérase et une paire d'amorces avec SEQ ID NO. 1 et 2, SEQ ID NO. 3 et SEQ ID NO. 4, SEQ ID NO. 5 et 6, SEQ ID NO. 1 et 7, SEQ ID NO. 1 et 8, SEQ ID NO. 1 et 9, SEQ ID NO. 1 et 10, SEQ ID NO. 11 et 12 ou un mélange des paires d'amorces avec SEQ ID NO. 1 et 7-10 pour amplifier des éléments répétitifs en ADN.

**Figur 1**

**Figur 2**

Figur 3

**Figur 4**

quantifizierbarer Bereich

**Figur 5**

```
For:  5'-AGGAGATCGAGACCATCCTGGC-3'    SEQ ID Nr. 1


A  G  G  A  G  A  T  C  G  A  G  A  C  C  A  T  C  C  T  G  G  C  ⎫
   A  A  C  A  T  G  G  A  T        G        G     T             ⎬  SEQ ID Nr.
            G     T                                              ⎭
            A                                                       11


Rev:  5'-TGGCGCAATCTCGGCTCACTG-3'    SEQ ID Nr. 2


T  G  G  C  G  C  A  A  T  C  T  C  G  G  C  T  C  A  C  T  G  ⎫
C     T  T  A  T  G        T     T  A  C              T  C    ⎬  SEQ ID Nr.
      C        G  T              G  T  T                      ⎭  12
                                 C
```

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4946778 A

- LU 92552

**In der Beschreibung aufgeführte Nicht-Patentliteratur**

- **MEHTA ; KEER.** *BioProcess International,* 2007, 44-58
- **HU, B. et al.** *J Pharm Biomed Anal.,* 2014, vol. 88, 92-95
- **PEPER, G. et al.** *J Pharm Biomed Anal,* 2014, vol. 100, 123-130
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KÖHLER ; MILSTEIN.** *Nature,* 1975, vol. 256, 494-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **J. A. WALKER et al.** Human DNA quantitation using Alu element - based polymerase chain reaction. *Anal. Biochem.,* 2003, vol. 315, 122-128


SEQUENCE LISTING

<110> Glycotope GmbH

<120> Verfahren zum Nachweis von Nukleinsaeuren in Proben mit biologischem Material

<130> GLY15154PCT

<150> LU92552
<151> 2014-09-22

<160> 12

<170> PatentIn version 3.5

<210> 1
<211> 22
<212> DNA
<213> artificial

<220>
<223> Alu-Forward primer

<400> 1
aggagatcga gaccatcctg gc        22

<210> 2
<211> 21
<212> DNA
<213> Artificial

<220>
<223> Alu-Reverse primer

<400> 2
tggcgcaatc tcggctcact g        21

<210> 3
<211> 22
<212> DNA
<213> artificial

<220>
<223> Alu-Forward primer

<400> 3
cgaggcgggt ggatcatgag gt        22

<210> 4
<211> 20
<212> DNA
<213> artificial

<220>
<223> Alu-Reverse primer

<400> 4
tctgtcgccc aggccggact        20

<210> 5
<211> 20
<212> DNA
<213> artificial

<220>
<223> Alu equivalent Forward primer

<400> 5
tggagagatg gctcgaggtt        20

<210> 6

<211> 21
<212> DNA
<213> artificial

<220>
<223> Alu equivalent Reverse primer

<400> 6
tggttgctgg gaattgaact c        21

<210> 7
<211> 21
<212> DNA
<213> artificial

<220>
<223> Alu-Reverse primer

<400> 7
tggtgcaatc tcggctcact g        21

<210> 8
<211> 21
<212> DNA
<213> artificial

<220>
<223> Alu-Reverse primer

<400> 8
tggcgcgatc tcggctcact g        21

<210> 9
<211> 21
<212> DNA
<213> artificial

<220>
<223> Alu-Reverse primer

<400> 9
tggcgctatc tcggctcact g        21

<210> 10
<211> 21
<212> DNA
<213> artificial

<220>
<223> Alu-Reverse primer

<400> 10
tggcgcaatc tcagctcact g        21

<210> 11
<211> 22
<212> DNA
<213> artificial

<220>
<223> Alu-Forward Primer

<220>
<221> misc
<222> (2)..(2)
<223> r is g or a

<220>
<221> misc
<222> (3)..(3)
<223> r is g or a

<220>
<221> misc
<222> (4)..(4)
<223> h is a or c

<220>
<221> misc
<222> (5)..(5)
<223> r is g or a

<220>
<221> misc
<222> (6)..(6)
<223> d is a, t or g

<220>
<221> misc
<222> (7)..(7)
<223> k is t or g

<220>
<221> misc_feature
<222> (8)..(8)
<223> n is a, c, g, or t

<220>
<221> misc
<222> (9)..(9)
<223> r is g or a

<220>
<221> misc
<222> (10)..(10)
<223> w is a or t

<220>
<221> misc
<222> (13)..(13)
<223> s is c or g

<220>
<221> misc
<222> (16)..(16)
<223> k is t or g

<220>
<221> misc
<222> (18)..(18)
<223> y is c or t

<400> 11
arrhrdknrw gascakcytg gc      22

<210> 12
<211> 21
<212> DNA
<213> artificial

<220>
<223> Alu-Reverse Primer

<220>
<221> misc
<222> (1)..(1)
<223> y is t or c

<220>
<221> misc
<222> (3)..(3)
<223> b is g, t or c

<220>
<221> misc
<222> (4)..(4)
<223> y is c or t

<220>
<221> misc
<222> (5)..(5)
<223> r is g or a

<220>
<221> misc
<222> (6)..(6)
<223> b is c, t or g

<220>
<221> misc
<222> (7)..(7)
<223> d is a, g or t

<220>
<221> misc
<222> (10)..(10)
<223> y is c or t

<220>
<221> misc
<222> (12)..(12)
<223> b is c, t or g

<220>
<221> misc_feature

<220>
<221> misc
<222> (13)..(13)
<223> n is a, c, g, or t

<220>
<221> misc
<222> (14)..(14)
<223> b is c, t or g

<220>
<221> misc
<222> (19)..(19)
<223> y is c or t

<220>
<221> misc
<222> (20)..(20)
<223> y is c or t

<400> 12
ygbyrbdaty tbnbctcayy g        21